# EUROPEAN PATENT SPECIFICATION

(11) **EP 4 362 855 B1**
(45) Date of publication and mention of the grant of the patent: **27.11.2024**
(21) Application number: 23772616.1
(22) Date of filing: 06.09.2023
(51) Int. Cl.: A61F 2/24

(54) **PROSTHETIC HEART VALVES**
HERZKLAPPENPROTHESEN
PROTHÈSES VALVULAIRES CARDIAQUES

(30) Priority: 06.09.2022 US 202263403965 P
(43) Date of publication of application: 08.05.2024
(60) Divisional application: 24206218.0
(73) Proprietor: The Global Heart Valve Innovation Center (Israel) Ltd., Or Yehuda 6037501 (IL)
(72) Inventor: IAMBERGER, Meni, 4464621 Kfar Saba (IL); HARARI, Boaz, 5592535 Ganey Tikva (IL)
(74) Representative: Evens, Paul Jonathan
(86) International application number: PCT/IL2023/050958
(87) International publication number: WO 2024/052909

(56) References cited:
- WO-A1-2021/178400
- CN-A- 112 603 598
- US-A1- 2019 224 008
- US-A1- 2020 069 417

## Description

### CROSS-REFERENCE TO RELATED APPLICATIONS

The present application claims priority from US Provisional Application 63/403,965, filed September 6, 2022, which is assigned to the assignee of the present application.

### FIELD OF THE INVENTION

The present invention relates to a prosthetic heart valve configured for transcatheter implantation, for replacement of a cardiac valve.

### BACKGROUND OF THE APPLICATION

Aortic regurgitation (AR), also known as aortic insufficiency (AI), is the leaking of the aortic valve of the heart that causes blood to flow in the reverse direction during ventricular diastole, from the aorta into the left ventricle. As a consequence, the cardiac muscle is forced to work harder than normal. Transcatheter aortic valve replacement (TAVR) is a treatment for aortic valve regurgitation and stenosis.

PCT Publication WO 2019/026059 to Hariton et al. describes a frame assembly including a tubular portion that defines a lumen along an axis; a plurality of arms coupled to the tubular portion at a first axial level, each of the arms extending radially outward from the tubular portion to a respective arm- tip; and a plurality of ventricular legs that are coupled to the tubular portion at a second axial level, and that extend radially outward from the tubular portion. A first sheet has a greater perimeter, and a smaller perimeter that defines an opening. The first sheet is stitched onto the plurality of arms, with the opening aligned with the lumen. Subsequently, an outer perimeter of a second sheet is stitched to the greater perimeter of the first sheet. Subsequently, the second sheet is everted by passing an inner perimeter of the second sheet around the arm-tips.

PCT Publication WO 2023/009379 to Bukin et al. describes annular frames for prosthetic heart valves that include commissure portions that are radially offset and/or are radially flexible relative to the rest of the frame. The commissure portions can extend radially outwardly from the rest of the frame such that they are positioned at a greater radius than the adjacent struts of the frame. The commissure portions can be cantilevered from the frame, such that only one axial end of the commissure portion is attached to the rest of the frame, allowing the commissure portions to flex radially inwardly and outwardly relative to the rest of the frame. Lateral arms can also couple upper ends of the commissure portions to adjacent apices to pull them in at larger diameters. The radial position of the commissure portions relative to the rest of the frame can affect the positioning of the leaflets and the effectiveness of the valve.

US Patent Application Publication 2022/0031454 to Straubinger et al. describes a method of placing an implant including a stent at a native heart valve site including orienting an expandable body of the stent such that a first open end of the stent is upstream a second open end of the stent relative to a direction of blood flow through a native heart valve. The native heart valve includes native leaflets and an annulus. The method also includes radially expanding a plurality of positioning arches positioned around an outer perimeter of the expandable body. The method additionally includes inserting the plurality of positioning arches respectively within a plurality of pockets defined by leaflets of the native heart valve and a heart structure from which the native leaflets extend. The method further includes radially expanding the first open end of the stent into a position upstream of the annulus and out of contact with nerve bundles.

US 2020/069417 relates to a prosthetic heart valve with paravalvular leak mitigation features, and discloses a prosthetic heart valve according to the pre-amble of appended independent claim 1.

US 2019/224008 relates to a frame for an implantable medical device and a method of manufacturing the frame.

### SUMMARY OF THE APPLICATION

In accordance with the present invention, there is provided a prosthetic heart valve as defined in appended independent claim 1. Embodiments of the present invention are defined in appended claims dependent on independent claim 1.

For some applications, a prosthetic valve is provided having a prosthetic valve frame assembly comprising an inner frame and an outer frame. The prosthetic valve is percutaneously deliverable to a native heart valve in a compressed state, and is expandable at the native valve to facilitate one-way inflow-to-outflow, upstream-to-downstream, fluid flow through the lumen in a proximal-to-distal direction. The prosthetic valve is typically designated for implantation at a native aortic valve of a patient. It is to be noted that the scope of the present invention includes implantation of the prosthetic valve at other native cardiac valves of the patient, e.g., a pulmonary valve, a mitral valve, or a tricuspid valve.

The outer frame of the prosthetic valve comprises at least four pairs, e.g., six pairs, of outer-frame struts and at least four, e.g., six, outer-frame junctions. These junctions function as tissue locators which locate and identify tissue at the downstream surface of the native valve (e.g., tissue at the downstream surface of the native cusp of the native leaflet). The pairs of outer-frame struts function as tissue anchors which anchor the native leaflet between the respective pair of outer-frame struts and the inner frame thereby securing the prosthetic valve at the native valve by sandwiching tissue of the native valve between the inner frame and the outer frame. The pairs of outer-frame struts prevent migration of the prosthetic valve in the proximal direction toward the left ventricle. Because the anchoring relies primarily on this sandwiching, the prosthetic valve substantially does not use outwardly-applied radial force to assist with the anchoring, i.e., does not require oversizing for anchoring. The sandwiching-based anchoring of the prosthetic valve may reduce the risk of requiring permanent pacemaker implantation, by reducing the mechanical pressure applied by the prosthetic valve on the conduction system adjacent to the aortic valve annulus.

(In some configurations, the prosthetic valve comprises an upstream sealing cuff, which may help prevent paravalvular leak (PVL). Although an upstream portion of the frame of the prosthetic may apply an outwardly-directed radial force to the surrounding aortic tissue, this radial force primarily provides sealing between the upstream sealing cuff and the surrounding aortic tissue, rather than materially assisting with anchoring.)

There is therefore provided, in accordance with an application of the present invention, a prosthetic heart valve configured for transcatheter implantation, the prosthetic valve including:
a prosthetic valve frame that surrounds a central longitudinal axis of the prosthetic valve to define a lumen along the axis when the prosthetic valve is in an expanded state; and
a plurality of prosthetic leaflets, disposed within the lumen, and arranged to facilitate one-way inflow-to-outflow fluid flow through the lumen in a proximal-to-distal direction when the prosthetic valve is in the expanded state,
wherein the prosthetic valve frame:
   includes a plurality of struts, and
   is shaped so as to define a plurality of intermediate nodes that are intermediate the proximal and distal ends of the prosthetic valve frame,
wherein a portion of the plurality of struts are intermediate struts,
wherein each intermediate node has at least four struts extending therefrom, the at least four struts including (a) a distally-extending pair of two struts of the at least four struts, and (b) a proximally-extending pair of two struts of the at least four struts,
wherein for each pair of circumferentially adjacent intermediate nodes:
   (i) a first distally-extending strut that extends from one intermediate node of the pair of intermediate nodes converges at a distal junction with (ii) a second distally-extending strut that extends from the other intermediate node of the pair of intermediate nodes,

   (i) a first proximally-extending strut that extends from the one intermediate node of the pair of intermediate nodes converges at a proximal junction with (ii) a second proximally-extending strut that extends from the other intermediate node of the pair of intermediate nodes, and

   (i) a first intermediate strut extends proximally from an intermediate location along the first distally-extending strut, and (ii) a second intermediate strut extends proximally from an intermediate location along the second distally-extending strut, the intermediate location along the first distally-extending strut being between the one intermediate node and the distal junction, and the intermediate location along the second distally-extending strut being between the other intermediate node and the distal junction,
wherein the first and second intermediate struts converge at an intermediate junction that points in a proximal direction,
wherein the intermediate junction is intermediate the distal junction and the proximal junction, and
wherein the proximal junction, the intermediate junction, and the distal junction are circumferentially aligned,
characterized in that:
   each intermediate node has at least six struts extending therefrom,
   the proximally-extending pair of the two struts defines a first proximally-extending pair of two struts of the at least six struts,
   the at least six struts include a second proximally-extending pair of two struts of the at least six struts,
   the proximal junction defines a first proximal junction, and
   for each pair of circumferentially adjacent intermediate nodes:
      (i) a third proximally-extending strut that extends from the one intermediate node of the pair of intermediate nodes converges at a second proximal junction with (ii) a fourth proximally-extending strut that extends from the other intermediate node of the pair of intermediate nodes,

      the third proximally-extending strut is proximal to the first proximally-extending strut,
      the fourth proximally-extending strut is proximal to the second proximally-extending strut,
      the second proximal junction is proximal to the first proximal junction, and
      the second proximal junction and first proximal junction are circumferentially aligned.

For some applications, when the prosthetic heart valve is in the expanded state, for each pair of circumferentially adjacent intermediate nodes:
a smaller rhombus of the prosthetic valve frame is bounded by (1) distal portions of the first and the second distally-extending struts, and (2) the first and the second intermediate struts, and
a larger rhombus of the prosthetic valve frame is bounded by (1) the first and the second distally-extending struts, and (2) the first and second proximally-extending struts, and
the smaller rhombus is within the larger rhombus.

For some applications, the first and the second intermediate struts have respective sequential longitudinal portions, and when the prosthetic valve is in the expanded state, the respective sequential longitudinal portions:
extend at an angle of 75 - 105 degrees from respective upstream sides of the first and the second distally-extending struts, respectively,
curve partially upstream and partially toward the respective closest intermediate nodes along respective first curved longitudinal portions of the first and the second intermediate struts, and
curve partially upstream and partially away from the respective closest intermediate nodes along respective second curved longitudinal portions of the first and the second intermediate struts.

For some applications, each of the first curved longitudinal portions has a length of 0.2 - 0.75 mm, and each of the second curved longitudinal portions has a length of 1 - 1.75 mm, the lengths measured along respective curved central longitudinal axes of the curved longitudinal portions.

For some applications:
each of the first curved longitudinal portions has a first length and each of the second curved longitudinal portions has a second length, the lengths measured along respective curved central longitudinal axes of the curved longitudinal portions, and
a ratio of the second length to the first length is 2 - 8.

For some applications, each of the first curved longitudinal portions has a first inner radius of curvature of 0.05 - 0.2 mm, and each of the second curved longitudinal portions has a second inner radius of curvature of 0.4 - 0.8 mm.

For some applications:
each of the first curved longitudinal portions has a first inner radius of curvature and each of the second curved longitudinal portions has a second inner radius of curvature, and
a ratio of the second inner radius of curvature to the first inner radius of curvature is 2- 10.

For some applications, the prosthetic valve frame has an overall height of 20-26 mm when the prosthetic valve is in the expanded state.

For some applications, when the prosthetic heart valve is in the expanded state, for each pair of circumferentially adjacent intermediate nodes:
a smaller rhombus of the prosthetic valve frame is bounded by (1) distal portions of the first and second distally-extending struts, and (2) the first and second intermediate struts,
a larger rhombus of the prosthetic valve frame is bounded by (1) the first and second distally-extending struts, and (2) the first and second proximally-extending struts, and
a largest rhombus of the prosthetic valve frame is bounded by (1) the first and second distally-extending struts, and (2) the third and fourth proximally-extending struts, and
the smaller rhombus is within the larger rhombus, and the larger rhombus is within the largest rhombus.

For some applications:
the portion of the plurality of struts includes a first portion of the plurality of struts,
the prosthetic valve frame includes a second portion of the plurality of struts,
the second portion of the plurality of struts are proximal struts, and
for each intermediate node:
   (i) a first proximal strut extends proximally from an intermediate location along a first strut of the second proximally-extending pair of the two struts of the at least six struts, and (ii) a second proximal strut extends proximally from an intermediate location along a second strut of the second proximally-extending pair of the two struts of the at least six struts, each intermediate location along the respective first and second struts of the second proximally-extending pair of two struts being between the intermediate node and respective proximal ends of each of the first and second struts of the second proximally-extending pair of two struts,

   the first and second proximal struts converge at a proximally-pointing junction of the first and second proximal struts,
   the proximally-pointing junction of the first and second proximal struts is circumferentially aligned with the intermediate node,
   and the proximally-pointing junctions are circumferentially offset with respect to the second proximal junction.

For some applications, the proximally-pointing junctions are longitudinally aligned with the second proximal junctions at the proximal end of the prosthetic valve frame.

For some applications:
each intermediate node has at least eight struts extending therefrom,
the distally-extending pair of two struts is a first distally-extending pair of two struts of the at least eight struts, and
for each pair of circumferentially adjacent intermediate nodes:
   (i) a third distally-extending strut that extends from the one intermediate node of the pair of intermediate nodes converges at the distal junction with (ii) a fourth distally-extending strut that extends from the other intermediate node of the pair of intermediate nodes,

   the third distally-extending strut is distal to the first distally-extending strut, and
   the fourth distally-extending strut is distal to the second distally-extending strut.

The present invention will be more fully understood from the following detailed description of applications thereof, taken together with the drawings, in which:

### BRIEF DESCRIPTION OF THE DRAWINGS

Figs. 1A-D are schematic illustrations of a prosthetic valve, and an inner frame of a frame assembly of the prosthetic valve, in accordance with some applications of the invention;
Figs. 2A-C are schematic illustrations of the prosthetic valve, and an outer frame of the frame assembly of the prosthetic valve, in accordance with some applications of the invention;
Figs. 3A-F are schematic illustrations of the frame assembly of the prosthetic valve, in accordance with some applications of the invention;
Figs. 4A-B are schematic illustrations of the inner frame in a compressed state, in accordance with some applications of the invention;
Figs. 5A-B are schematic illustrations of the outer frame in a compressed state, in accordance with some applications of the invention;
Figs. 6A-B are schematic illustrations of frame assembly in a compressed state, in accordance with some applications of the invention;
Figs. 7A-F are schematic illustrations of implantation of the prosthetic valve at a native aortic valve of a patient;
Fig. 8 is a schematic illustration of an inner frame of another prosthetic valve, in accordance with an application of the present invention;
Fig. 9 is a schematic illustration of an outer frame of the prosthetic valve of Fig. 8, in accordance with an application of the present invention;
Fig. 10 is a schematic illustrations of a prosthetic valve frame assembly of the prosthetic valve of Figs. 8 and 9, in accordance with some applications of the invention;
Figs. 11A-B are schematic illustrations of the inner frame of the prosthetic valve of Fig. 8, including a skirt and prosthetic leaflets, in accordance with respective applications of the present invention;
Fig. 11C is a schematic illustration of the prosthetic valve frame assembly of the prosthetic valve of Fig. 10, including the skirt and the prosthetic leaflets of Figs. 11, in accordance with an application of the present invention;
Figs. 12A-H are schematic illustrations of additional configurations of the inner frame of Fig. 8, in accordance with respective applications of the present invention;
Figs. 13A-B are schematic illustrations of additional configurations of the outer frame of Figs. 2A-C, in accordance with respective applications of the present invention; and
Fig. 14 is a schematic illustration of another outer frame of a prosthetic valve.

### DETAILED DESCRIPTION OF EMBODIMENTS

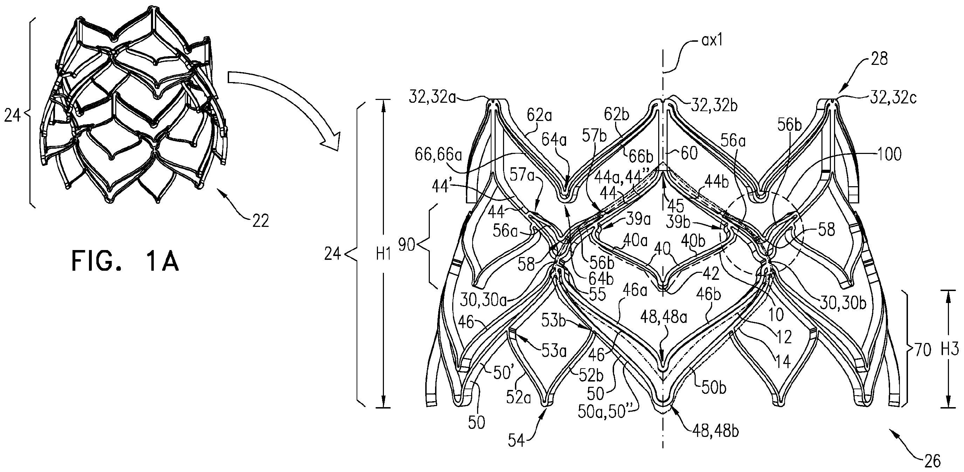

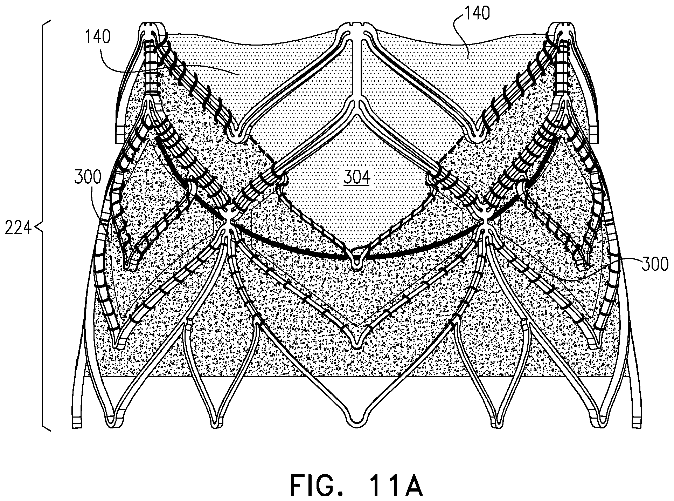

Reference is made to Figs. 1A-D, 2A-C, and 3A-F, which are schematic illustrations of a prosthetic valve 22 comprising a prosthetic valve frame assembly 21 comprising an inner frame 24 and an outer frame 80, in accordance with some applications of the invention. The prosthetic valve is for use at a native heart valve of a subject - typically the aortic valve. The prosthetic valve has a compressed state for minimally-invasive (typically transluminal, e.g., transfemoral) delivery, and an expanded state into which prosthetic valve 22 is transitioned at the native heart valve, and in which prosthetic valve 22 provides prosthetic valve functionality. Prosthetic valve 22 comprises prosthetic valve frame assembly 21, a skirt 300 comprising flexible sheeting (such as such as described hereinbelow with reference to Figs. 11A-C for prosthetic valve 222), and a valve member comprising prosthetic leaflets 140.

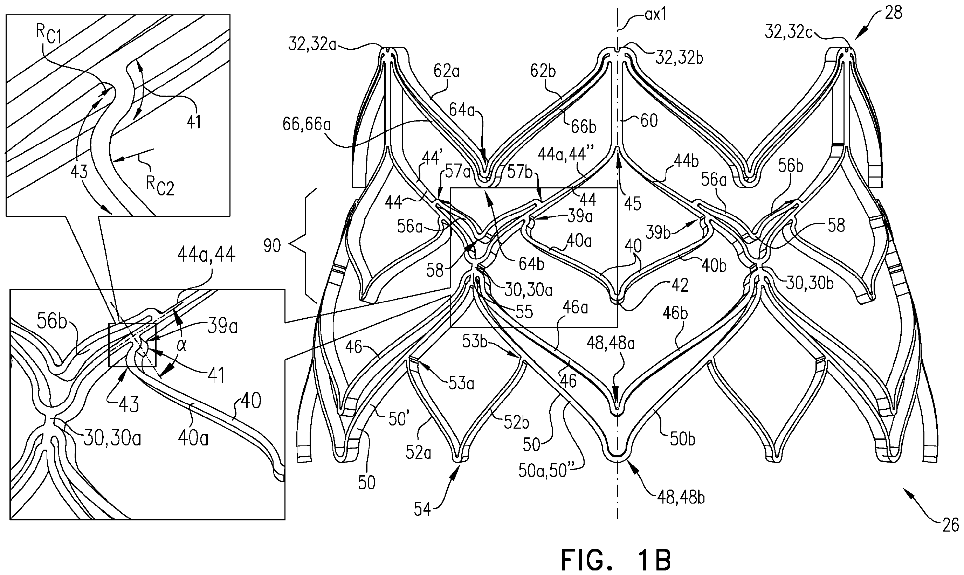

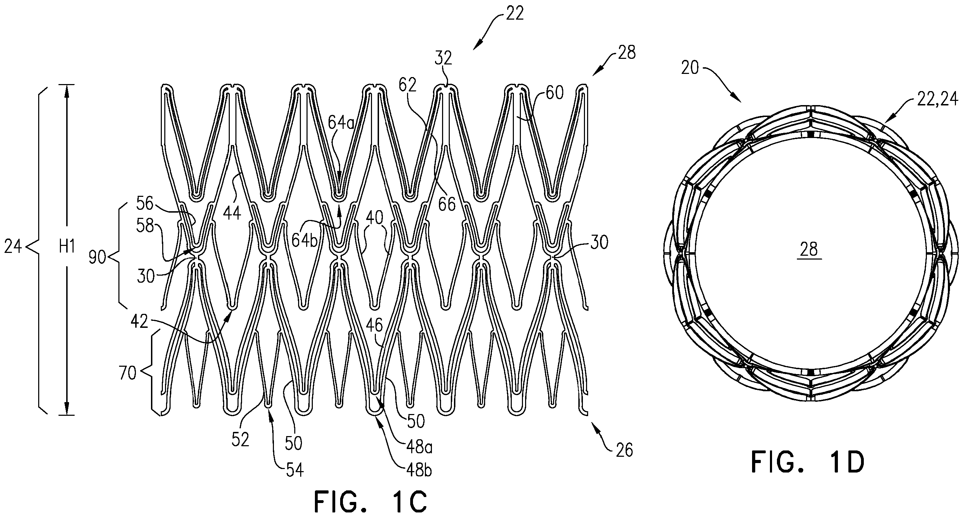

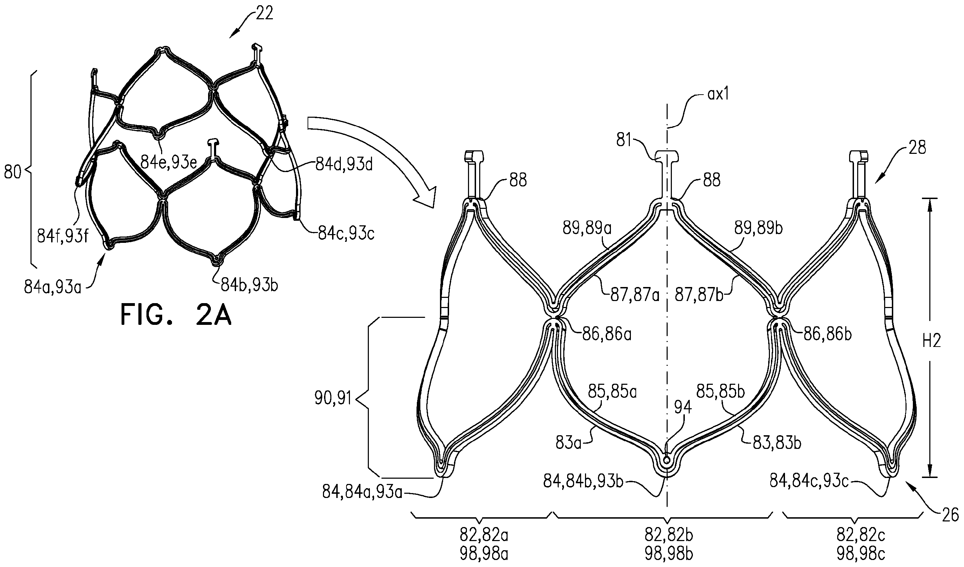

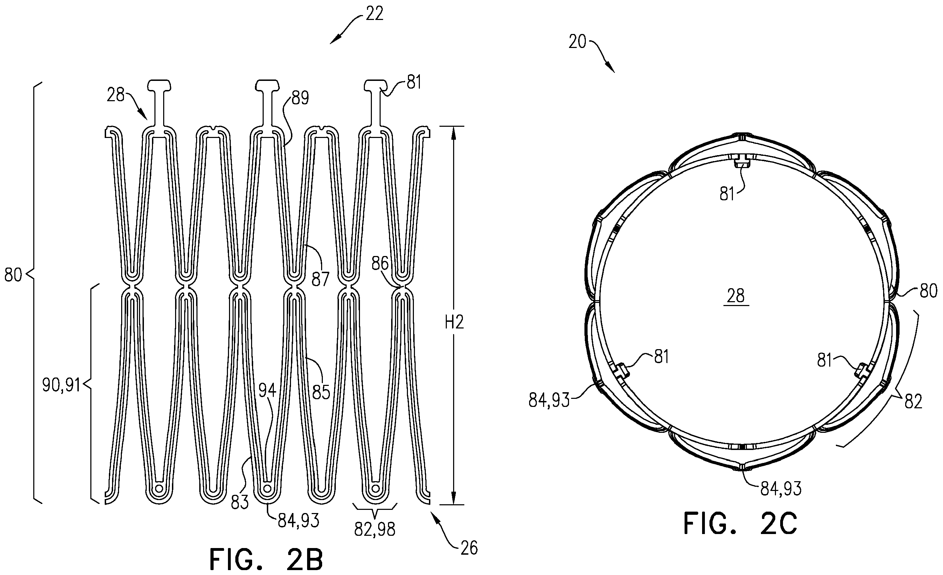

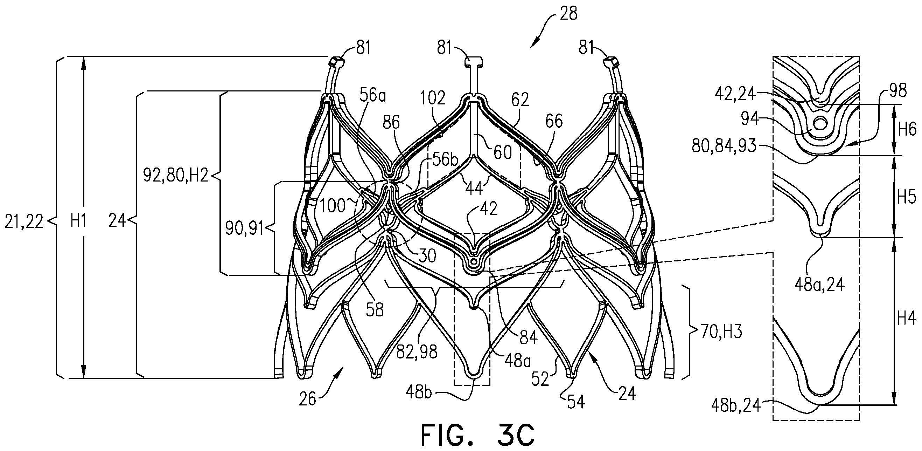

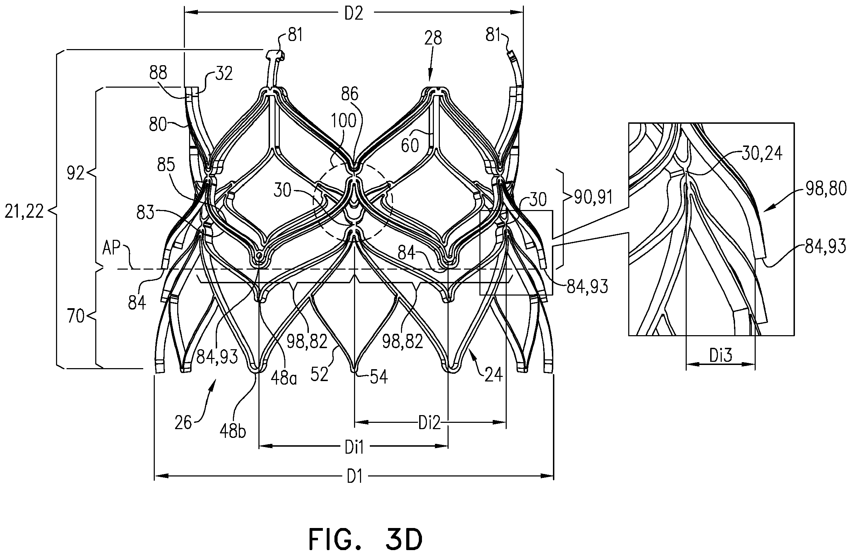

Figs. 1A-D, 2A-C, and 3A-F show prosthetic valve 22 and frames thereof in the expanded state. For clarity of illustration, Fig. 1A shows inner frame 24 of frame assembly 21 alone in an isometric and side view, and Fig. 1B shows inner frame 24 alone in a side view with fewer reference numerals for the sake of clarity. For clarity of illustration, the enlarged portion of Fig. 1A and the entirely of Fig. 1B show a circumferential half of inner frame 24. Fig. 1C shows a flat roll-out view of inner frame 24. Fig. 1D shows a top, outflow view of inner frame 24. Fig. 2A shows outer frame 80 of frame assembly 21 alone in an isometric and side view. Fig. 2B shows a flat roll-out view of outer frame 80. Fig. 2C shows a top, outflow view of outer frame 80. For clarity of illustration, the enlarged portion of Fig. 2A shows a circumferential half of outer frame 80, and the entireties of Figs. 3C and 3D show a circumferential half of enlarged portion of prosthetic valve 22.

For clarity of illustration, Figs. 1A-3D omit prosthetic leaflets 140, although prosthetic valve 22 comprises prosthetic leaflets 140. Optionally, prosthetic valve 22 further comprises skirt 300 and/or upstream sealing cuff 310, such as described hereinbelow with reference to Figs. 11A-C for prosthetic valve 222.

Prosthetic valve 22, and each of inner frame 24 and outer frame 80, has an upstream, proximal end 26, a downstream, distal end 28, and defines a central longitudinal axis ax1 therebetween. Frame assembly 21 comprises a prosthetic valve frame that comprises a valve body (which is a generally tubular portion), and is shaped to define a lumen through the valve body from its upstream end to its downstream end. The valve body circumscribes axis ax1, and thereby defines the lumen along the axis. Throughout this application, including the specification and the claims, unless stated otherwise, "upstream" and "downstream," e.g., with respect to the ends of prosthetic valve 22, are defined with respect to the longitudinal axis of valve 22, by the orientation and functioning of the prosthetic leaflets, which facilitate one-way upstream-to-downstream fluid flow through the lumen in a proximal-to-distal direction. Throughout this application, including the specification and the claims, unless stated otherwise, "proximal" and "distal," e.g., with respect to the ends of prosthetic valve 22, are defined with respect to the longitudinal axis of valve 22, by the orientation and functioning of the prosthetic leaflets, which facilitate one-way upstream-to-downstream fluid flow through the lumen in a proximal-to-distal direction.

Figs. 2A-C show outer frame 80. Outer frame 80 comprises appendages 81 configured to reversibly couple prosthetic valve 22 to a delivery tool. Frame assembly 21 has an overall height H1 that is 20-26 mm, e.g., 22.0-24.83 mm. H1 does not include appendages 81. H1 also typically represents the height of inner frame 24. As such, relative to other aortic valves in the field of aortic valve replacement, the present invention provides a shorter overall height H1 of prosthetic valve 22 as compared to conventional transcatheter prosthetic aortic valves. This shortened overall height H1 is hypothesized by the inventors to minimize or even eliminate interference with openings 124 and 126 of coronary arteries 123 and 125, respectively, and to shorten the supra-annular portion 92 of prosthetic valve 22.

Figs. 1A-D show inner frame 24. It is to be noted that inner frame 24 is typically used in combination with outer frame 80 as shown in Figs. 2A-C and, in general, herein. It is to be noted however, that the scope of the present invention includes the use of inner frame 24 (1) on its own itself, i.e., without an outer frame or (2) in combination with any other suitable valve frame, e.g., a suitable additional outer frame or a suitable additional inner frame.

Prosthetic valve inner frame 24 comprises a plurality of struts, comprising a biocompatible material, e.g., Nitinol. Inner frame 24 is shaped so as to define a plurality of intermediate nodes 30 that are intermediate the proximal and distal ends 26 and 28 of inner frame 24. Prosthetic valve inner frame 24 is also shaped so as to define a plurality of distal end nodes 32 that are at distal end 28 of inner frame 24. Intermediate nodes 30 are circumferentially offset with respect to distal end nodes 32.

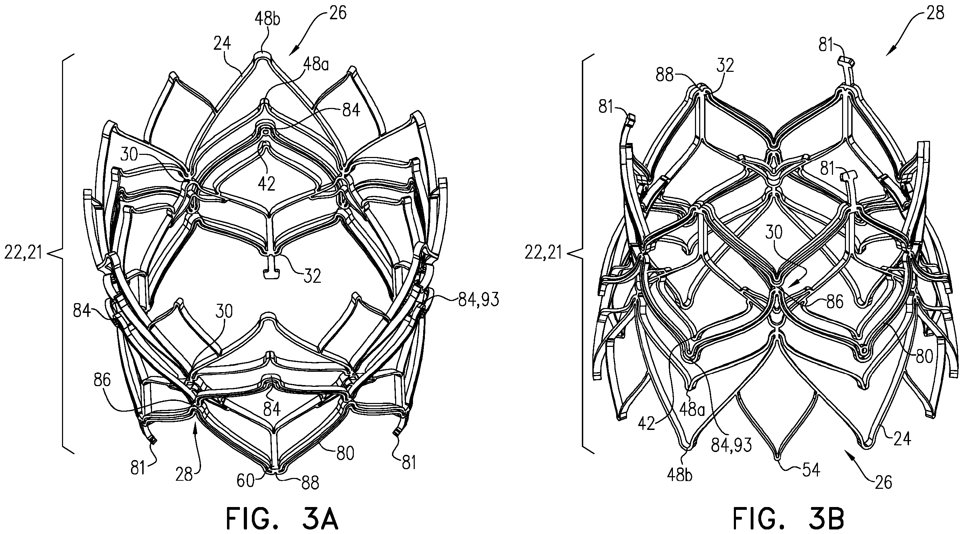

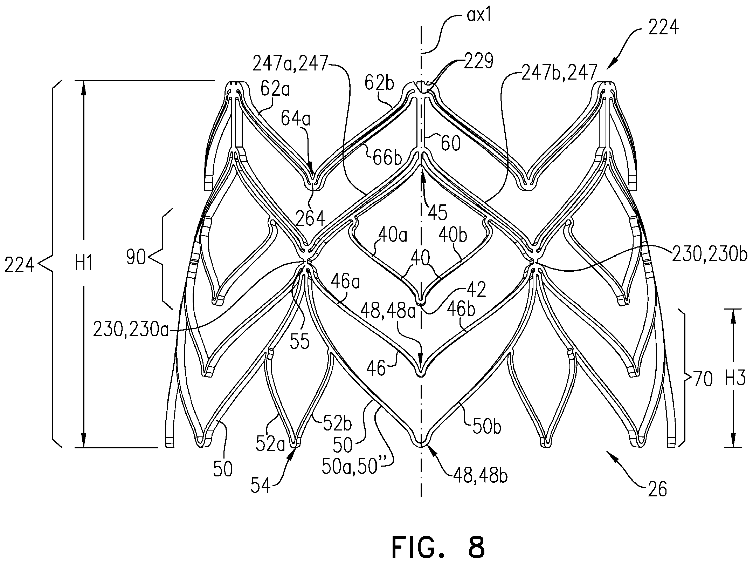

A first portion of the plurality of struts of inner frame 24 are intermediate struts 40. Each intermediate node 30 has at least six struts (e.g., exactly six struts, such as shown in Figs. 1A-D and 3A-D, or exactly eight struts, such as shown in Figs. 8, 10, 11A-C, and 12, described hereinbelow). The struts extending from each intermediate node 30 include (a) a distally-extending pair of two struts 44 of the at least six struts, and (b) a proximally-extending pair of two struts 46 of the at least four struts. Optionally, each of intermediate nodes 30 is shaped so as to define a waist axially separating the ends of the distally-extending pair of two struts 44 from the ends of the proximally-extending pair of two struts 46; for example, the waist may have a length of 0.1 - 0.3 mm, such as 0.12 - 0.2 mm, e.g., 0.15 mm.

Reference is made to Figs. 1A-C. For each pair of circumferentially adjacent intermediate nodes 30, for example:
- (i) a first distally-extending strut 44a that extends from one intermediate node 30a of the pair of intermediate nodes 30 converges at a distal junction 45 with (ii) a second distally-extending strut 44b that extends from the other intermediate node 30b of the pair of intermediate nodes 30,
- (i) a first proximally-extending strut 46a that extends from the one intermediate node 30a of the pair of intermediate nodes 30 converges at a proximal junction 48a with (ii) a second proximally-extending strut 46b that extends from the other intermediate node 30b of the pair of intermediate nodes 30, and
- (i) a first intermediate strut 40a extends proximally from an intermediate location 39a along the first distally-extending strut 44a, and (ii) a second intermediate strut 40b extends proximally from an intermediate location 39b along the second distally-extending strut 44b. The intermediate location 39a along the first distally-extending strut 44a is between the one intermediate node 30a and distal junction 45. The intermediate location 39b along the second distally-extending strut 44b is between the other intermediate node 30b and the distal junction 45.

The first intermediate strut 40a and second intermediate strut 40b converge at an intermediate junction 42 that points in a proximal direction. The intermediate junction 42 is intermediate the distal junction 45 and the proximal junction 48a. The intermediate junction 42, the distal junction 45, and the proximal junction 48a are circumferentially aligned. Typically, intermediate struts 40 and intermediate junctions 42 are disposed in an anchoring region 90 of inner frame 24 and of prosthetic valve 22 and provide increased radial strength and stiffness to valve 22 at anchoring region 90.

As labeled in Fig. 1A, for some applications, for each pair of circumferentially adjacent intermediate nodes 30:
- a smaller rhombus 10 (or similar shape) of the prosthetic valve inner frame 24 is bounded by (1) distal portions of the first and second distally-extending struts 44a and 44b, and (2) the first and second intermediate struts 40a and 40b,
- a larger rhombus 12 (or similar shape) of prosthetic valve inner frame 24 is bounded by (1) the first and second distally-extending struts 44a and 44b, and (2) the first and second proximally-extending struts 46a and 46b, and
- the smaller rhombus 10 is within the larger rhombus 12.

Alternatively, inner frame 24 is not shaped so as to define first intermediate strut 40a and second intermediate strut 40b.

Reference is still made to Figs. 1A-C. For applications in which each intermediate node 30 has at least six struts extending therefrom (as shown):
- the proximally-extending pair of two struts 46 is a first proximally-extending pair of two struts 46 of the at least six struts,
- the at least six struts comprise a second proximally-extending pair of two struts 50 of the at least six struts, and
- the proximal junction defines a first proximal junction 48a.

For each pair of circumferentially adjacent intermediate nodes 30:
- (i) a third proximally-extending strut 50a that extends from the one intermediate node 30a of the pair of intermediate nodes 30 converges at a second proximal junction 48b with (ii) a fourth proximally-extending strut 50b that extends from the other intermediate node 30b of the pair of intermediate nodes 30,
- the third proximally-extending strut 50a is proximal to the first proximally-extending strut 46a,
- the fourth proximally-extending strut 50b is proximal to the second proximally-extending strut 46b,
- the second proximal junction 48b is proximal to the first proximal junction 48a, and
- the first and second proximal junctions 48a and 48b are circumferentially aligned.

As shown in Figs. 1A-C, each of first proximal junction 48a, its corresponding second proximal junction 48b, its corresponding intermediate junction 42, its corresponding distal end node 32, and its corresponding distal junction 45 are circumferentially aligned. For some applications, as shown, (1) third and fourth proximally-extending struts 50a and 50b are connected to (2) first and second proximally-extending struts 46a and 46b at a connector 55. As shown, connector comprises a vertical metal element. It is to be noted that (1) struts 50a and 50b and (2) struts 46a and 46b may be connected using any suitable connector.

For some applications, such as shown in Figs. 1A-B, for each pair of circumferentially adjacent intermediate nodes 30:
- a smaller rhombus 10 of the prosthetic inner valve inner frame 24 is bounded by (1) distal portions of the first and second distally-extending struts 44a and 44b, and (2) the first and second intermediate struts 40a and 40b,
- a larger rhombus 12 of the prosthetic inner valve inner frame 24 is bounded by (1) the first and second distally-extending struts 44a and 44b, and (2) the first and second proximally-extending struts 46a and 46b, and
- a largest rhombus 14 (or similar shape) of the prosthetic inner valve inner frame 24 is bounded by (1) the first and second distally-extending struts 44a and 44b, and (2) the third and fourth proximally-extending struts 50a and 50b.

The smaller rhombus 10 is within the larger rhombus 12, and the larger rhombus 12 is within the largest rhombus 14. As such, inner frame 24 defines a rhombus-within-rhombus section.

A second portion of the plurality of struts of the prosthetic valve inner frame 24 are proximal struts 52. Typically, proximal struts 52 are disposed at the inflow, proximal end 26 of prosthetic valve inner frame 24 and provide increased strength and rigidity to a subannular portion 70 of the native aortic valve. This increased strength and rigidity provided by proximal struts in helpful in minimizing or eliminating paravalvular leaks. Additionally, proximal struts 52, in combination with the first and second proximal junctions 48a and 48b, which are longitudinally close together (as is described hereinbelow with reference to Fig. 3C), and provide additional increased strength and rigidity to subannular portion 70 of the native aortic valve.

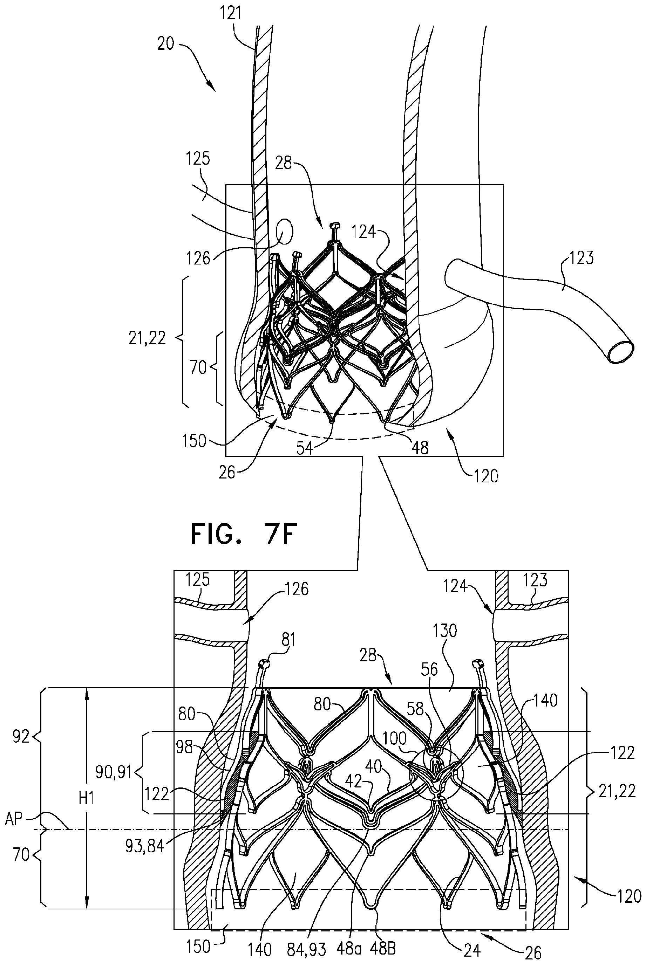

Typically, subannular portion 70 is covered by a covering, e.g., a fabric, a polyester such a Dacron(TM), velour cloth, an inflatable cuff that is inflatable either with blood or fluid, and/or any other suitable material. Typically, the covering is a fabric, e.g., a polyester such a Dacron(TM). This covering 150 is described hereinbelow with reference to Fig. 7F. Typically, subannular portion 70 has a height H3 of 5.4-6.5 mm e.g., 5.4 mm.

As labeled in Figs. 1A-B, for each intermediate node 30:
- (i) a first proximal strut 52a extends proximally from an intermediate location 53a along a first strut 50' of the second proximally-extending pair of two struts 50 of the at least six struts that extend from intermediate node 30, and (ii) a second proximal strut 52b extends proximally from an intermediate location 53b along a second strut 50" of the second proximally-extending pair of two struts 50 of the at least six struts that extend from intermediate node 30.
- Each intermediate location 53a and 53b along respective first and second struts 50' and 50" of the second pair of two struts 50 of proximally-extending struts is between the intermediate node 30 and respective proximal ends of each of the first and second struts 50' and 50" of the second pair of two struts 50 of proximally-extending struts.
- The first and second proximal struts 52a and 52b converge at a proximally-pointing junction 54 of the first and second proximal struts 52a and 52b.
- The proximally-pointing junction 54 of the first and second proximal struts 52a and 52b is circumferentially aligned with the intermediate node 30. Each proximally-pointing junction 54 is circumferentially offset with respect to its neighboring second proximal junction 48b.
- The proximally-pointing junctions 54 are typically but not necessarily longitudinally aligned with the second proximal junctions 48b at proximal end 26 of the prosthetic valve inner frame 24.

Reference is still made to Figs. 1A-B. A third portion of the plurality of struts of the prosthetic valve inner frame 24 are reinforcing struts 56. As described hereinabove, each intermediate node 30 has at least four struts extending therefrom: (a) a distally-extending pair of two struts 44 of the at least four struts, and (b) a proximally-extending pair of two struts 46 of the at least four struts. A first reinforcing strut 56a extends proximally from an intermediate location 57a along a first strut 44' of the pair of distally-extending struts 44, and (ii) a second reinforcing strut 56b extends proximally from an intermediate location 57b along a second strut 44" of the pair of distally-extending struts 44. Each intermediate location 57a and 57b along respective first and second struts 44' and 44" of the pair of distally-extending struts 44 is between the intermediate node 30 and respective distal ends of each of the first and second struts 44' and 44" of the pair of distally-extending struts 44. First and second reinforcing struts 56a and 56b converge at a proximally-pointing junction 58 of the first and second reinforcing struts 56a and 56b. (1) The proximally-pointing junction 58 of the first and second reinforcing struts 56a and 56b, and (2) its corresponding intermediate node 30 are circumferentially aligned. Additionally, (1) the proximally-pointing junction 58 of the first and second reinforcing struts 56a and 56b, (2) its corresponding intermediate node 30, and (3) its corresponding proximally-pointing junction 54 are circumferentially aligned.

Reference is still made to Figs. 1A-B. Typically, reinforcing struts 56a and 56b are at a position of the inner frame 24 at which adjacent to the commissures of the prosthetic leaflets 140 of prosthetic valve 22. As shown, each of reinforcing struts 56a and 56b is coupled to a respective distally-extending struts 44. As described hereinbelow with reference to Fig. 3C, leaflet connectors 102 connect prosthetic leaflets 140 of prosthetic valve 22 to inner frame 24 by being sutured (i.e., stitched) to respective distally-extending struts 44 and to other struts. Leaflet connector 102 form the commissures of prosthetic leaflets 140 of prosthetic valve. Since reinforcing struts 56a and 56b are coupled to respective distally-extending struts 44, reinforcing struts 56a and 56b reinforce that position and thereby reinforce the adjacent prosthetic commissures of the prosthetic valve 22. Additionally, reinforcing struts 56a and 56b additionally strengthen, stiffen, and provide additional rigidity to each of distally-extending struts 44. As such, each pair of reinforcing struts 56a and 56b create a respective reinforced zone 100 of prosthetic valve 22. As shown, in zone 100 prosthetic valve 22 defines a semi-double-strutted region of prosthetic valve 22. The semi-double-strutted region is particularly appropriate in such an area of increased density due to the presence of leaflet connectors 102 of prosthetic valve 22. Thus, in order to accommodate for the decrease in space due to the leaflet connectors 102 of the prosthetic leaflets 140, the smaller reinforcing struts 56 as compared to other struts of valve 22 are used in zone 100. Typically, each reinforcing strut 56 is 20-80%, such as 30-60%, a length of each intermediate strut 40. Additionally, the relatively smaller lengths of each reinforcing strut 56 provides increased rigidity to the strut as compared to a longer strut. It is hypothesized by the inventors that reinforcing struts 56 are particularly suited to accommodate radial forces at the left ventricular outflow tract.

Reference is still made to Figs. 1A-B. Each distal end node 32 has at least two struts extending therefrom which comprise a distal-end-node-proximally-extending pair of two struts 62. For each pair of circumferentially adjacent distal end nodes 32:
- a first distal-end-node-proximally-extending strut 62a that extends from one distal end node 32a of the pair of distal end nodes 32 converges at a first outflow junction 64a with (ii) a second distal-end-node-proximally-extending strut 62b that extends from the other distal end node 32b of the pair of distal end nodes 32, and
- (1) the first outflow junction 64a, (2) the proximally-pointing junction 58 of the first and second reinforcing struts 56a and 56b, and (3) the corresponding intermediate node 30 are circumferentially aligned.

Each distal end node 32 has at least four struts extending therefrom. The distal-end-node-proximally-extending pair of two struts 62 defines a first distal-end-node-proximally-extending pair of two struts 62, and the at least four struts of the distal end node 32 comprise a second distal-end-node-proximally-extending pair of two struts 66.

Reference is still made to Figs. 1A-B. For each pair of circumferentially adjacent distal end nodes 32:
- a third distal-end-node-proximally-extending strut 66a that extends from the one distal end node 32a of the pair of distal end nodes 32 converges at a second outflow junction 64b with (ii) a third distal-end-node-proximally-extending strut 66b that extends from the other distal end node 32b of the pair of distal end nodes, and
- first and second outflow junctions 64a and 64b are circumferentially aligned.

As shown in Figs. 1A-B, the proximally-pointing junction 58 of the first and second reinforcing struts 56a and 56b, (2) its corresponding intermediate node 30, (3) its corresponding proximally-pointing junction 54 are circumferentially aligned, and its corresponding the first and second outflow junctions 64a and 64b are circumferentially aligned.

As shown in Figs. 1A-B, each distal end node 32 has at least five struts extending therefrom. The at least five struts comprise a vertical strut 60 which extends proximally from the distal end node 32 and toward distal junction 45 and is disposed between (1) the first and third distal-end-node-proximally-extending struts 62a and 66a and (2) the second and fourth distal-end-node-proximally-extending struts 62b and 66b.

The struts of the distal, outflow portion of inner frame 24 can be described as arranged in a double-strut formation in which pairs of two inner-frame distal struts (i.e., distal-end-node-proximally-extending pair of two struts 62 and second distal-end-node-proximally-extending pair of two struts 66) are arranged alongside each other. The double-strut formation at the outflow, distal end 28 of inner frame 24 define the distal perimeter of inner frame 24 and are arranged in an undulating or zigzag pattern as a series of ascending and descending struts between circumferentially-adjacent distal end nodes 32. It is hypothesized by the inventors that the double-strut formation provides radial strength and rigidity to the distal, outflow portion of inner frame 24, and of prosthetic valve 22. Additionally, the double-strut formation of inner frame 24 enhances the strength of inner frame 24 such that it does not deform in response to hydrodynamic forces and other forces acted upon prosthetic valve 22 by the native tissue.

As shown in Figs. 1A-B, second proximal junctions 48b extend further radially away from axis ax1 than do distal end nodes 32 from axis ax1 such that the proximal end 26 of inner frame 24 is wider than the distal end 28 of inner frame 24. The diameter of inner frame 24 gradually decreases from proximal end 26 to distal end 28 in a tapering manner.

As shown in Figs. 1A-B and labeled in Fig. 1B, first and second intermediate struts 40a and 40b have respective sequential longitudinal portions. For some applications, when the prosthetic valve is in the expanded state, the respective sequential longitudinal portions:
- extend at an angle α (alpha) of 75 - 105 degrees, e.g., 85 - 95 degrees, such as 90 degrees, from respective upstream sides of first and second distally-extending struts 44a and 44b, respectively,
- curve partially upstream and partially toward respective closest intermediate nodes 30a and 30b (along respective first curved longitudinal portions 41 of first and second intermediate struts 40a and 40b), and
- curve partially upstream and partially away from respective closest intermediate nodes 30a and 30b (along respective second curved longitudinal portions 43 of first and second intermediate struts 40a and 40b).

Optionally, only a single one of first and second intermediate struts 40a and 40b of each pair of circumferentially adjacent intermediate nodes 30 has the shape described immediately above.

For each of intermediate struts 40a and 40b, an end of first curved longitudinal portion 41 and a beginning of second curved longitudinal portion 43 are defined by a point of inflection between first curved longitudinal portion 41 and second curved longitudinal portion 43. For each of intermediate struts 40a and 40b, an end of second curved longitudinal portion 43 is defined by either (a) a point of inflection along the intermediate strut or (b) a point at which the intermediate strut becomes straight.

For example, each first curved longitudinal portion 41 may have a length of at least 0.2 mm (e.g., at least 0.25 mm), no more than 0.75 mm (e.g., no more than 0.6 mm), and/or 0.2 - 0.75 mm (e.g., 0.25 - 0.6 mm). Alternatively or additionally, for example, each second curved longitudinal portion 43 may have a length of at least 1 mm (e.g., at least 1.1 mm), no more than 1.75 mm (e.g., no more than 1.5 mm), and/or 1 - 1.75 mm (e.g., 1.1 - 1.5 mm). These lengths are measured along respective curved central longitudinal axes of the curved longitudinal portions. Further alternatively or additionally, for example, a ratio of the length of each second curved longitudinal portion 43 to the length of each first curved longitudinal portion 41 may be 1.5 - 10, e.g., 2-8, such as 2 - 6.

For example, each first curved longitudinal portion 41 may have a first inner radius of curvature R_{C1} of at least 0.05 mm (e.g., at least 0.06 mm), no more than 0.2 mm (e.g., no more than 0.16 mm), and/or 0.05 - 0.2 mm (e.g., 0.06 - 0.16 mm). Alternatively or additionally, for example, each second curved longitudinal portion 43 may have a second inner radius of curvature R_{C2} of at least 0.4 mm (e.g., at least 0.5 mm), no more than 0.8 mm (e.g., no more than 0.7 mm), and/or 0.4 - 0.8 mm (e.g., 0.5 - 0.7 mm). Further alternatively or additionally, for example, a ratio of the second inner radius of curvature R_{C2} to the first inner radius of curvature R_{C1} may be 2 - 10, e.g., 3-9.

The shapes of the above-described curved portions of first and second intermediate struts 40a and 40b may help (a) provide a low crimping profile and/or (b) create an optimal curvature for these struts such that when these struts meet at intermediate junction 42, these struts can be stitched to the upstream part of the scallop of the prosthetic valve without having to apply tension to or fold the scallop (this stitching is shown in Figs. 11A-C for prosthetic valve 222).

Figs. 2A-C show prosthetic valve outer frame 80 of prosthetic valve 22. For some applications, such as shown, outer frame 80 comprises appendages 81 configured to reversibly couple prosthetic valve 22 to a delivery tool. Outer frame 80 Frame has a height H2 that is 12-17 mm, e.g., 13.3-15.9 mm. Height H2 does not include appendages 81. For some applications, outer frame 80 has a wall thickness that is smaller than the wall thickness of inner frame 24 which decreases the crimping profile of prosthetic valve 22 when inside the delivery tube. Alternatively, for some applications, inner frame 24 comprises appendages 81, either instead of outer frame 80, or in addition to outer frame 80 (configurations not shown). Whether inner frame 24 or outer frame 80 comprises appendages 81, the appendages extend distally downstream beyond distal end 28 of the respective frame, i.e., are not considered part of the respective frame, and thus are not included in the respective height of the respective frame.

Prosthetic valve outer frame 80 comprises a plurality of struts, comprising a biocompatible material, e.g., Nitinol. Outer frame 80 is shaped so as to define a plurality of intermediate nodes 86 that are intermediate the proximal and distal ends 26 and 28 of outer frame 80. Prosthetic valve outer frame 80 is also shaped so as to define a plurality of distal end nodes 88 that are at distal end 28 of outer frame 80. Intermediate nodes 86 are circumferentially offset with respect to distal end nodes 88.

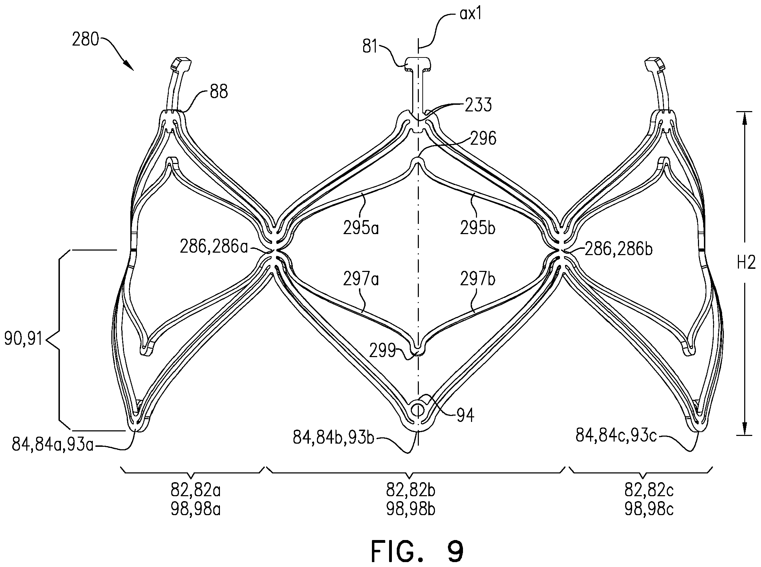

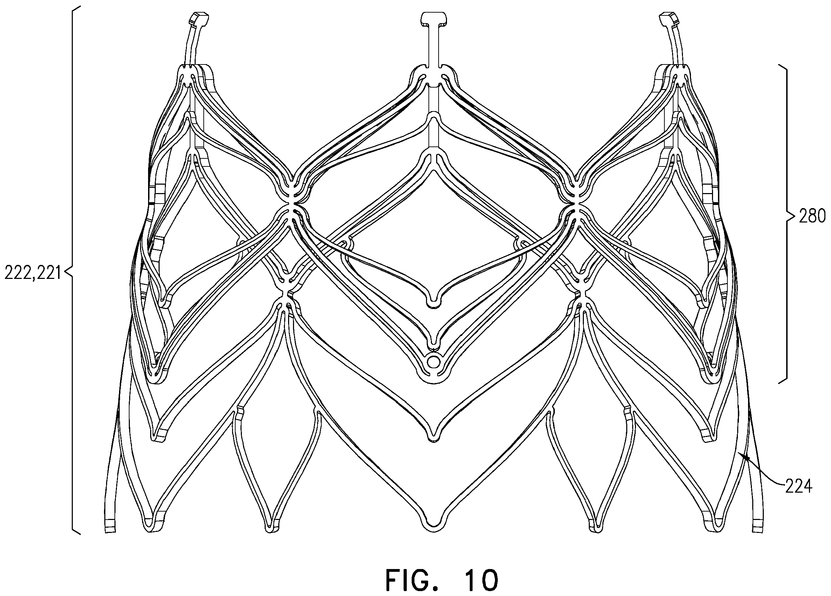

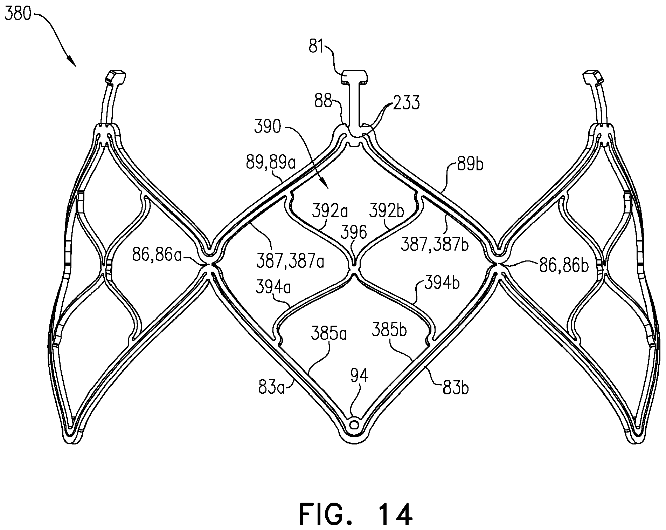

Reference is made to Fig. 2A. Each intermediate node 86 has at least four struts extending therefrom, e.g., at least eight struts (e.g., exactly eight struts), such as shown in Figs. 2A-B and 3A-D (and Figs. 14, 21, 22, and 23, described hereinbelow), or at least 12 struts (e.g., exactly 12 struts), such as shown in Figs. 9 and 10, described hereinbelow. The struts extending from each intermediate node 86 include (a) a distally-extending pair of two struts 87 (87a and 87b) of the at least four struts, and (b) a proximally-extending pair of two struts 85 (85a and 85b) of the at least four struts.

For embodiments in which each intermediate node 86 has at least eight struts extending therefrom, such as shown, the struts extending from each intermediate node include (a) a first distally-extending pair of the two struts 87 (87a and 87b) of the at least eight struts, (b) a second distally-extending pair of the two struts 89 (89a and 89b) of the at least eight struts, (c) a first proximally-extending pair of two struts 85 (85a and 85b) of the at least eight struts, and (d) a second proximally-extending pair of two struts 83 (83a and 83b) of the at least eight struts.

Reference is still made to Fig. 2A. For each pair of circumferentially adjacent intermediate nodes 86 of outer frame 80, for example:
- (i) a first distally-extending distal strut 87a that extends from one intermediate node 86a of the pair of intermediate nodes 86 converges at distal end node 88 with (ii) a second distally-extending distal strut 87b that extends from the other intermediate node 86b of the pair of intermediate nodes 86, and
- (i) a first proximally-extending proximal strut 85a that extends from the one intermediate node 86a of the pair of intermediate nodes 86 converges at a first outer-frame proximal junction 94 with (ii) a second proximally-extending proximal strut 85b that extends from the other intermediate node 86b of the pair of intermediate nodes 86.

Also, as shown in Fig. 2A, for each pair of circumferentially adjacent intermediate nodes 86 of outer frame 80, for example:
- (i) a third distally-extending distal strut 89a that extends from one intermediate node 86a of the pair of intermediate nodes 86 converges at distal end node 88 with (ii) a fourth distally-extending distal strut 89b that extends from the other intermediate node 86b of the pair of intermediate nodes 86, and
- (i) a third proximally-extending proximal strut 83a that extends from the one intermediate node 86a of the pair of intermediate nodes 86 converges at a second outer-frame proximal junction 84 with (ii) a fourth proximally-extending proximal strut 83b that extends from the other intermediate node 86b of the pair of intermediate nodes 86.

Reference is still made to Fig. 2A. Typically, the struts of outer frame 80 are arranged in a multiple-strut formation, such as a double-strut formation. In the double-strut formation:
- pairs of two outer-frame distal struts (i.e., two struts 87 and two struts 89) are arranged alongside each other. The double-strut formation at the outflow, distal end 28 of outer frame 80 define the distal perimeter of outer frame 80 and are arranged in an undulating or zigzag pattern as a series of ascending and descending struts between circumferentially-adjacent distal end nodes 88. It is hypothesized by the inventors that the double-strut formation provides radial strength and rigidity to the distal, outflow portion of outer frame 80, and of prosthetic valve 22, and
- pairs of two outer-frame proximal struts (i.e., two struts 83 and two struts 85) are arranged alongside each other. The double-strut formation at the inflow, proximal end 26 of outer frame 80 define the proximal perimeter of outer frame 80 and are arranged in an undulating or zigzag pattern as a series of ascending and descending struts between circumferentially-adjacent outer-frame proximal junctions 84. It is hypothesized by the inventors that the double-strut formation provides radial strength and rigidity to the inflow, proximal portion of outer frame 80, and of prosthetic valve 22.

Typically, as shown in Figs. 2A-B, the entire outer frame 80 is arranged in a multiple-strut formation, such as a double-strut formation.

Alternatively, the multiple-strut formation is a triple-strut formation, such as described hereinbelow with reference to Fig. 9.

The multiple-strut formation, such as the double-strut formation, of outer frame 80 enhances the strength of outer frame 80 such that it does not deform in response to hydrodynamic forces and other forces acted upon prosthetic valve 22 by the native tissue.

Typically, the pairs of two outer-frame proximal struts (i.e., two struts 83 and two struts 85) and outer-frame proximal junctions 84 and 94 are disposed in an anchoring region 90 of outer frame 80 and of prosthetic valve 22 and provide increased radial strength and stiffness to valve 22 at anchoring region 90. Typically, outer-frame proximal struts 83 and 85 and outer-frame proximal junctions 84 and 94 are covered in foam, e.g., polyurethane.

Outer-frame proximal junctions 84 extend further radially away from axis ax1 than do distal end nodes 88 from axis ax1 such that the proximal end 26 of outer frame 80 is wider than the distal end 28 of outer frame 80.

Reference is made to Figs. 2A-B. Each outer-frame proximal junction 84 is circumferentially aligned with a corresponding distal end node 88. Outer-frame proximal junctions 84 are longitudinally aligned. As shown, outer frame 80 comprises at least four outer-frame proximal junctions 84a, 84b, 84c, and 84d. For example, such as shown, outer frame 80 comprises at least six outer-frame proximal junctions 84a, 84b, 84c, 84d, 84e, and 84f. outer-frame proximal junctions 84. Each outer-frame proximal junction 84 (and for some applications, together with junctions 94) defines a tissue position locator 93 which is configured to abut against and locate tissue at the outflow surface of the native cusp of the native leaflet of the native aortic valve. Each locator 93 is formed by respective pairs 82 of outer-frame struts which converge at respective outer-frame junctions (i.e., 84 and/or 94) that point in the proximal direction. The plurality of pairs 82 of outer-frame struts are circumferentially disposed so as to engage an outflow surface of the native aortic valve. The locators 93 are typically not specifically aligned with cusps or commissures of the native aortic valve, although they may become aligned by chance during deployment. Alternatively, prosthetic valve 22 is rotated during delivery such that exactly two locators 93 are disposed within each cusp of the native aortic valve.

As shown in Figs. 2A-B, each pair 82 of outer-frame struts comprises at least two proximally-extending proximal struts 83 from respective circumferentially-adjacent intermediate nodes 86. For applications in which outer frame 80 is arranged in a multiple-strut formation (e.g., a double-strut formation, such as shown), each pair 82 of outer-frame struts comprises at least (1) two proximally-extending proximal struts 83 from respective circumferentially-adjacent intermediate nodes 86, and (2) two proximally-extending proximal struts 85 from respective circumferentially-adjacent intermediate nodes 86. As shown, outer frame 80 comprises at least four pairs 82 of outer-frame struts. For example, such as shown, outer frame 80 comprises at least six pairs 82 of outer-frame struts, as can be seen in the isometric view of Fig. 2A, and in Figs. 2B-C; in the enlarged portion of Fig. 2A, which shows a circumferential half of outer frame 80, three of the six pairs can be seen and are labeled 82a, 82b, and 82c.

Prosthetic valve 22 thus defines at least four, e.g., six as shown in Figs. 2A-C, outer-frame tissue anchors 98 which each comprise (1) a pair 82 of outer-frame struts 83 (and for some applications a double-strut pair of outer-frame struts 83 and 85), and (2) an outer-frame proximal junction 84 (and for some applications, together with junctions 94) that defines a tissue position locator 93. Unlike conventional prosthetic aortic valves which typically comprise three locators and three tissue anchors, prosthetic valve 22 of the present invention comprises at least four, e.g., six, locators 93 and anchors 98 so as to increase the imaging visibility and to increase the likelihood of proper alignment, anchoring, and sealing of prosthetic valve 22 with respect to the native aortic valve.

The pairs 82 of outer-frame struts and outer-frame proximal junctions 84 and 94 defining locators 93 are disposed in a tissue-locating region 91 of outer frame 80 and of prosthetic valve 22 and provide increased radial strength and stiffness to valve 22 at tissue-locating region 91.

Each distal end node 88 has at least two struts extending therefrom which comprise first and second distal-end-node-proximally-extending distal struts 89. As shown, each distal end node 88 has at least four struts extending therefrom which comprise first and second distal-end-node-proximally-extending distal struts 89 and third and fourth distal-end-node-proximally-extending distal struts 87.

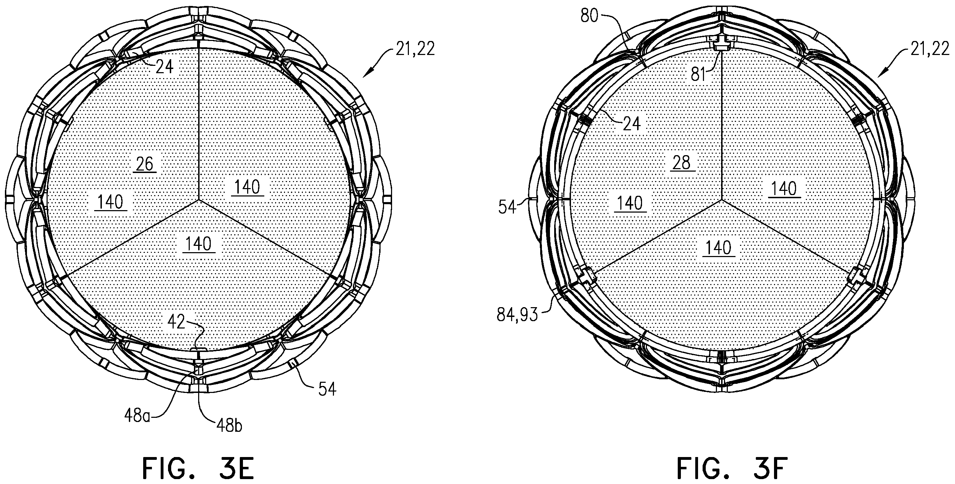

Fig. 3A is an isometric view of frame assembly 21 from an inflow-outflow direction. Fig. 3B is an isometric view of frame assembly 21 from an outflow-inflow direction. Figs. 3C-D are side views of frame assembly 21 at different rotations. Fig. 3E is a bottom, inflow view of frame assembly 21. Fig. 3F is a top, outflow view of frame assembly 21.

Fig. 3C shows the relative distances between respective junctions of inner frame 24 and outer frame 80 of prosthetic valve 22. As shown in the enlarged portion of Fig. 3C, (1) intermediate junction 42 of inner frame 24, (2) first outer-frame proximal junction 94 of outer frame 80, (3) second outer-frame proximal junction 84 (which defines a portion of tissue position locator 93) of outer frame 80, (4) first proximal junction 48a of inner frame 24, and (5) second proximal junction 48b of inner frame 24, all point in the proximal direction and are circumferentially aligned. Such relative positioning of the junctions of both inner frame 24 and outer frame 80 decreases overall gap space between metal components of prosthetic valve 22 and increases overall radial forces of prosthetic valve 22 against tissue of the native aortic valve. This particular configuration of multiple circumferentially-aligned and longitudinally disposed junctions helps prosthetic valve 22 withstand the high pressures associated with the left ventricular outflow tract and the aortic valve. Prosthetic valve 22 defines a height H4 between (a) the proximal end of second proximal junction 48b, and (b) the proximal end of first proximal junction 48a of between 4.5-8.0 mm, e.g., 5.2-6.4 mm. Prosthetic valve 22 defines a height H5 between (a) the proximal end of first proximal junction 48a, and (b) the proximal end of second outer-frame proximal junction 84 of between 2.2-3.0 mm, e.g., 2.5-2.6 mm. Prosthetic valve 22 defines a height H6 between (a) the proximal end of second outer-frame proximal junction 84, and (b) the proximal end of intermediate junction 42 of inner frame 24 of between 1.2-2.2 mm, e.g., 1.6-1.9 mm.

Thus, prosthetic valve 22 is configured in a manner in which:
- the inner frame 24 is shaped so as to define a plurality of pairs of inner-frame struts which converge at respective inner-frame junctions 42, 48a, and 48b that point in the proximal direction, the plurality of pairs of inner-frame struts being circumferentially disposed so as to engage an inflow surface of the native aortic valve of the patient,
- the outer frame 80 is shaped so as to define a plurality of pairs 82 of outer-frame struts which converge at respective outer-frame junctions 84 that point in the proximal direction, the plurality of pairs 82 of outer-frame struts being circumferentially disposed so as to engage an outflow surface of the native aortic valve,
- each one of the outer-frame proximal junctions 84 is circumferentially aligned with a corresponding inner frame junctions 42, 48a, and 48b,
- the plurality of pairs of inner-frame struts and the plurality of pairs 82 of outer-frame struts are configured to engage leaflets of the native valve between corresponding pairs of inner-frame struts and pairs 82 of outer-frame struts, and
- the outer frame 80 is shaped so as to define at least four pairs 82 of outer-frame struts and at least four outer-frame junctions 84.

Fig. 3C, as well as other figures herein, additionally shows the relationship between intermediate node 86 of outer frame 80 and intermediate node 30 of inner frame 24, and in particular with reinforcing struts 56 and proximally-pointing junction 58. As such, each pair of reinforcing struts 56a and 56b, in combination with junction 58 and nodes 30 and 86 create a respective reinforced zone 100 of prosthetic valve 22. Typically, reinforced zone 100 reinforces the adjacent prosthetic commissures of the prosthetic valve 22 are coupled to frame assembly 21 of prosthetic valve 22.

Fig. 3C also shows in phantom a leaflet connector 102 which is typically directly coupled to inner frame 24, e.g., typically by suturing, i.e., stitching. Leaflet connector 102 is typically sutured (i.e., stitched) to (1) respective portions of pairs of distal-end-node-proximally-extending struts 66 and/or to respective portions of pairs of distal-end-node-proximally-extending struts 62, and (2) to respective portions of pairs of distally-extending struts 44. For some applications, leaflet connector 102 is sutured (i.e., stitched) to vertical strut 60. It is to be noted that the scope of the present invention includes coupling of the leaflet connectors 102 to the outer frame 80 in addition to the inner frame 24.

Fig. 3D shows prosthetic valve 22 having an outer diameter D1 at inflow, proximal end 26 of 26-36 mm, e.g., 28.6-35.2 mm. Prosthetic valve 22 has an outer diameter D2 at outflow, distal end 28 of 23-32 mm, e.g., 24.82-30.82 mm. Prosthetic valve 22 defines a distance Di1 between the outer-frame proximal junctions 84 of circumferentially-adjacent outer-frame tissue anchors 98 of 12-19 mm, e.g., 14.3-17.3 mm Prosthetic valve 22 defines a distance Di2 between circumferentially-adjacent intermediate nodes 30 of inner frame 24 of 11-18 mm, e.g., 13.0-15.9 mm. Distance Di2 is also the distance between circumferentially-adjacent intermediate nodes 86 of outer frame 80.

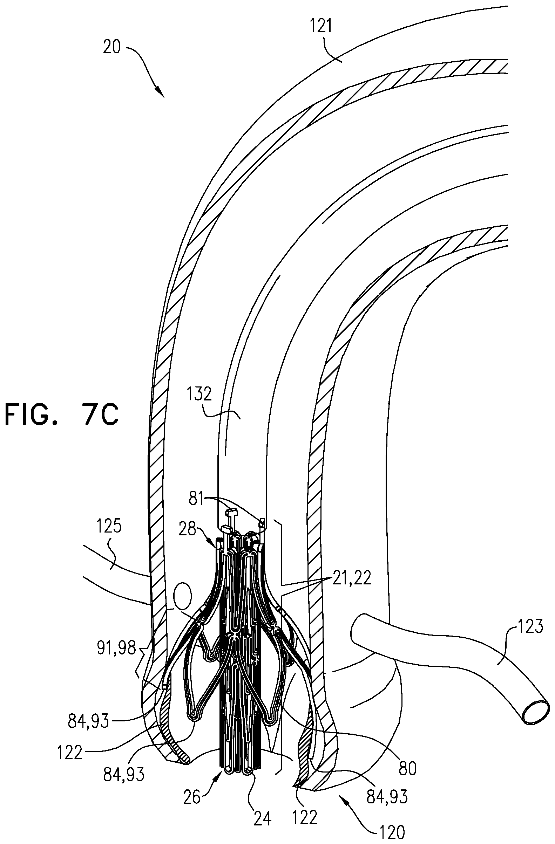

Outer frame 80 is directly coupled to inner frame 24 at outflow, distal end 28, specifically, distal end nodes 88 of outer frame 80 are directly coupled to distal end nodes 32 of inner frame 24. Typically, outer frame 80 is sutured (i.e., stitched) to inner frame 24. For some applications, outer frame 80 is mechanically coupled to inner frame 24 in any suitable manner, e.g., welding or by coupling pins or other apparatus. As shown in Fig. 3D, the coupling of outer frame 80 to inner frame 24 is at the outflow, distal end 28 so as to enable increased radial flexibility of outer frame 80 as it progresses proximally from distal end 28. Additionally, the coupling of outer frame 80 to inner frame 24 is at the outflow, distal end 28 so as to enable increased radial expansion of out-frame tissue anchors 98 and locators 93, as shown hereinbelow with reference to Fig. 7C. The struts of outer frame 80 at anchoring region 90 and tissue-locating region 91 are not directly connected to inner frame 24 and thereby, these struts are capable of (1) flexing radially outwardly enough to accommodate leaflet tissue, while (2) maintaining sufficient rigidity in order to compress the native leaflets between (1) the outer-frame struts 83 and 85 at anchoring region 90 of outer frame 80 (2) and the inner frame struts at anchoring region 90. For some applications, outer frame 80 is directly coupled to inner frame 24 only at distal end 28 of prosthetic valve 22. As such, as shown in Fig. 3D, prosthetic valve 22 defines a distance Di3 between (1) outer-frame proximal junction 84 and tissue position locator 93, and (2) an outer surface of inner frame 24 of 0.8-2.2 mm, e.g., 1.1-1.5 mm.

As shown in Fig. 3D, an annular plane AP is drawn in phantom so as to show the relative position of prosthetic valve 22 in the native aorta. Anchoring region 90, tissue-locating region 91, and supra-annular portion 92 are typically disposed distal to annular plane AP and subannular portion 70 is typically disposed proximal to annular plane AP.

Figs. 3E-F show prosthetic valve 22 in respective blood inflow and blood outflow views. As shown, prosthetic valve 22 comprises three prosthetic leaflets 140 which arranged to facilitate one-way inflow-to-outflow fluid flow through the lumen of prosthetic valve 22 in a proximal-to-distal direction. Figs. 3E-F are shown without a covering of prosthetic valve 22. For example, prosthetic leaflets 140 may comprise pericardium, such as bovine or porcine pericardium, or a synthetic polymer, as is known in the prosthetic valve art.

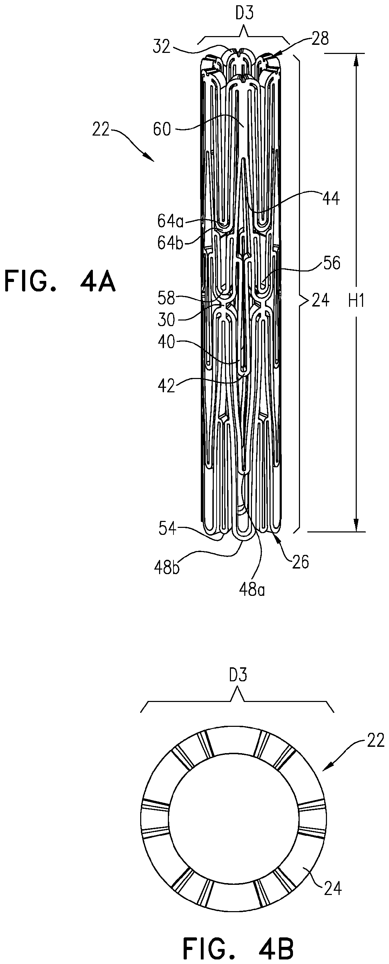

Reference is now made to Figs. 4A-B which are schematic illustrations of inner frame 24 of prosthetic valve 22 in a crimped configuration for delivery through a delivery tube, in accordance with some applications of the invention. In the crimped configuration, inner frame 24 has a crimped, outer diameter D3 of 4.5-6.5 mm, e.g., 5.5-6.0 mm.

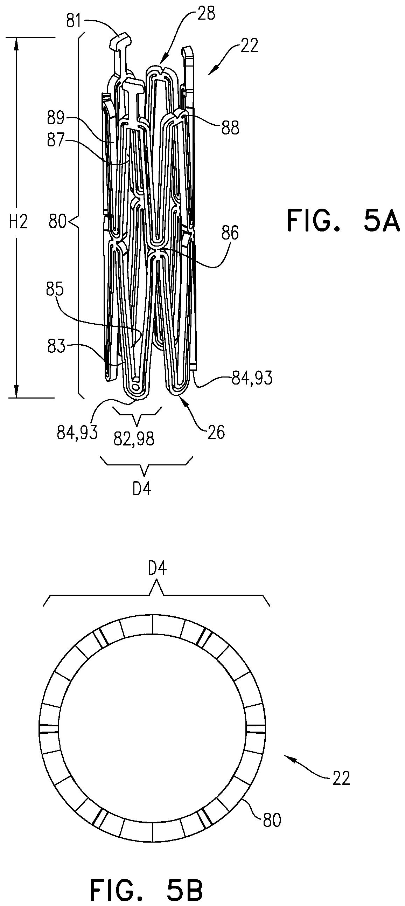

Reference is now made to Figs. 5A-B which are schematic illustrations of outer frame 80 of prosthetic valve 22 in a crimped configuration for delivery through a delivery tube, in accordance with some applications of the invention. In the crimped configuration, outer frame 80 has a crimped, outer diameter D4 of 5.0-7.5 mm.

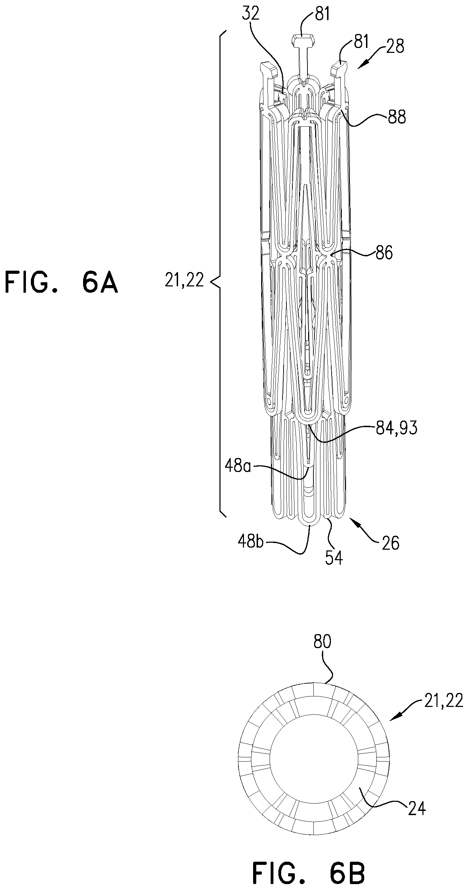

Reference is now made to Figs. 6A-B hich are schematic illustrations of prosthetic valve frame assembly 21 comprising inner frame 24 and outer frame 80 of prosthetic valve 22 in a crimped configuration for delivery through a delivery tube, in accordance with some applications of the invention. Outer frame 80 surrounds inner frame 24.

For some applications, one or more capsules (not shown) surround all or a portion of outer frame 80. Additionally, one or more capsules (not shown) surround all or at least a proximal portion of inner frame 24 to allow for controlled release of prosthetic valve 22 in the native aortic valve. Each capsule is independently controllable for sequential expanding of respective portions of valve frame assembly 21 for optimal controlled, sequential delivery of prosthetic valve 22.

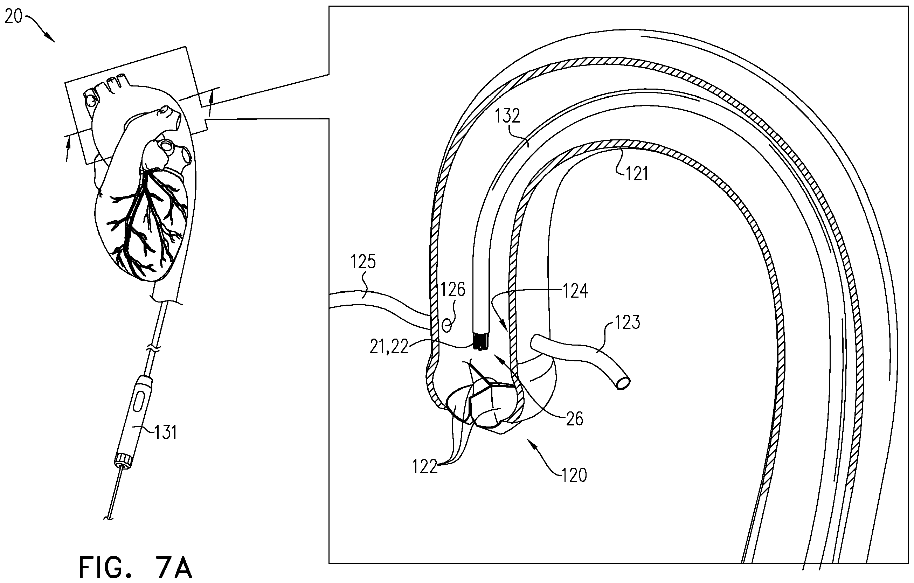

Reference is now made to Figs. 7A-F, which are schematic illustrations of a method for transcatheter implantation of prosthetic valve 22 at a native aortic valve 120 of a patient,. Also shown is a system 20, which comprises prosthetic valve 22 and a delivery system 131 comprising a delivery tube 132, housing prosthetic valve 22 in its crimped (compressed) configuration, is used to advance prosthetic valve 22 to aortic valve 120 using a transaortic approach, as shown. It is to be noted that tube 132 may be inserted through a blood vessel in the leg (transfemoral), through a large blood vessel in the heart (transaortic) or through the bottom tip of the heart (transapical).

In Fig. 7A, once the end of tube 132 is positioned within aorta 121 downstream of aortic valve 120, the upstream proximal end 26 of prosthetic valve 22 is exposed from within tube 132. As such, proximal end 26 is exposed in the ascending aorta upstream of the aortic sinuses and upstream of openings 124 and 126 of coronary arteries 123 and 125, respectively, and upstream of downstream surfaces of the native cusps of native leaflets 122 of native aortic valve 120.

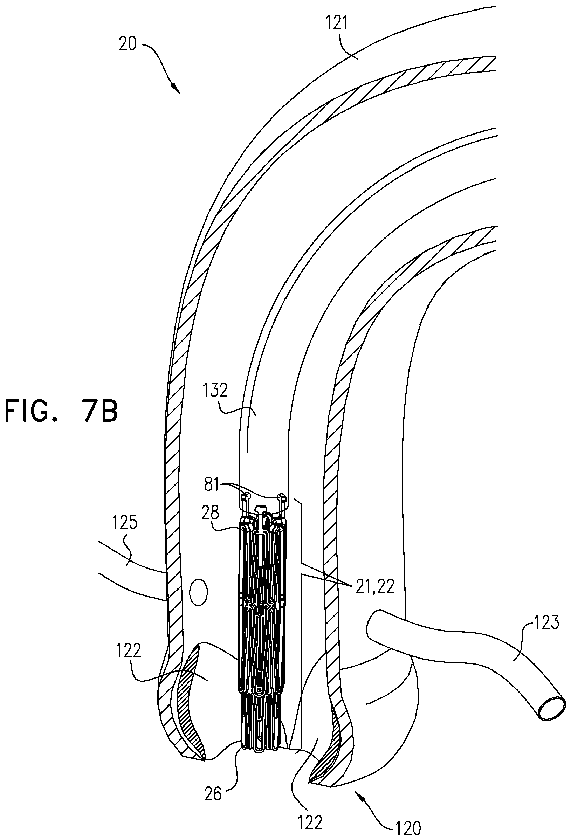

In Fig. 7B, prosthetic valve 22 is advanced proximally and out of tube 132 and is moved axially proximally and distally to properly position prosthetic valve 22 within native aortic valve 120. It is to be noted that one or more delivery capsules (not shown) surrounds prosthetic valve 22 such that it remains in the crimped configuration. As shown, prosthetic valve 22 is reversibly coupled to delivery tube 132 using appendages 81.

In Fig. 7C, a proximal portion of outer frame 80 of prosthetic valve 22 is exposed from within the one or more delivery capsules (not shown) such that outer-frame tissue anchors 98 radially expand. Collectively, outer-frame tissue anchors form a downstream flange. Under imaging guidance, prosthetic valve 22 is moved such that outer-frame tissue anchors 98 and tissue position locators 93 are positioned at a downstream surface of cusps of native leaflets 122 of the native aortic valve 120. In such a manner, prosthetic valve 22 is delivered to native aortic valve 120 in a manner in which native leaflets 122 are disposed between outer-frame tissue anchors 98 and the outer surface of inner frame 24. Locating region 91 of outer frame 80 is thus appropriately positioned at the downstream surface of the native aortic valve 120. At this stage of expanding and positioning of outer-frame tissue anchors 98 and locators 93, inner frame 24 remains disposed within the one or more capsules (not shown) such that inner frame 24 remains in the crimped configuration. Additionally, at this stage, distal end 28 remains crimped due to the appendages 81 being surrounded by the end of delivery tube 132.

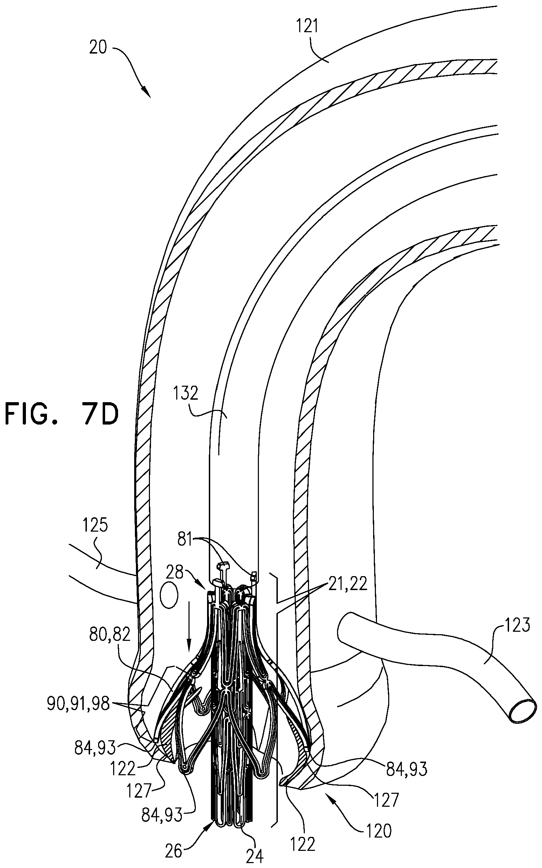

Typically, the outer-frame tissue anchors 98 and locators 93 radially expand from within a delivery capsule by retracting the delivery capsule distally. For some applications, outer-frame tissue anchors 98 and locators 93 are allowed to radially expand at a site downstream of native aortic valve 120. In such an application, prosthetic valve 22 is then pushed in the upstream direction so as to position outer-frame tissue anchors 98 and locators 93 at the downstream surface of native aortic valve 120, as described hereinbelow with reference to Fig. 7D. For some applications, delivery tube 132 is advanced upstream such that its distal end is within the left ventricle, and outer-frame tissue anchors 98 and locators 93 are allowed to radially expand at a site upstream of native aortic valve 120, e.g., within the left ventricle. In such an application, prosthetic valve 22 is then (1) retracted in the downstream direction so as to draw the outer-frame tissue anchors 98 and locators 93 downstream through the native aortic valve 120, and subsequently, (2) prosthetic valve 22 is pushed in the upstream direction so as to position outer-frame tissue anchors 98 and locators 93 at the downstream surface of native aortic valve 120, as described hereinbelow with reference to Fig. 7D.

In Fig. 7D, prosthetic valve 22 is pushed by delivery tube 132 in the upstream, proximal direction with respect to the native aortic valve such that outer-frame tissue anchors 98 and tissue position locators 93 are appropriately positioned at the downstream surface of cusps of native leaflets 122 of the native aortic valve 120. Additionally, position locators 93 and proximal junctions 84 are pushed in the upstream, proximal direction until they abut the surface and align with the downstream surfaces of cusps 127 of native aortic valve 120. That is, junctions 84 touch the base of leaflet 122 at the junction between the atrial wall and the leaflet 122. For some applications, junctions 84 touch the base of leaflet 122 in the middle of cusp 127. Under imaging guidance, e.g., fluoroscopy, the appropriate alignment and position of junctions 84 at cusps 127 is confirmed by locators 93 which typically, but not necessarily comprise radiopaque markers. For some applications, a first portion of locators 93 and/or of first outer-frame proximal junction 94 comprise radiopaque markers, while a second portion of locators 93 and/or of first outer-frame proximal junction 94 do not comprise radiopaque markers, and thus function as tissue anchors. The first portion of locators 93 and/or of first outer-frame proximal junction 94 are locators 93 and/or of first outer-frame proximal junction 94 which are present at the commissures of prosthetic valve 22. These first portion of locators 93 and/or of first outer-frame proximal junction 94 comprise the radiopaque markers which help align the commissures of prosthetic valve 22 with the native commissures of the native aortic valve 120. In addition to aiding the positioning of prosthetic valve 22 valve by facilitating imaging, the first portion of locators 93 and/or of first outer-frame proximal junction 94 also provide tactile feedback during the positioning of the valve. The second portion of locators 93 and/or of first outer-frame proximal junction 94 is for providing tactile feedback during positioning and for providing and anchoring.

Outer-frame tissue anchors 98 and locators 93 provide mechanical anchoring and prevent migration of prosthetic valve 22 in the upstream direction away from native aortic valve 120 and toward the left ventricle.

At this stage of (1) confirming proper positioning of tissue anchors 98 with the downstream surface of native leaflets 122, and (2) proper alignment of locators 93 and with cusps 127, inner frame 24 remains disposed within the one or more capsules (not shown) such that inner frame 24 remains in the crimped configuration. Additionally, at this stage, distal end 28 remains crimped due to the appendages 81 being surrounded by the end of delivery tube 132.

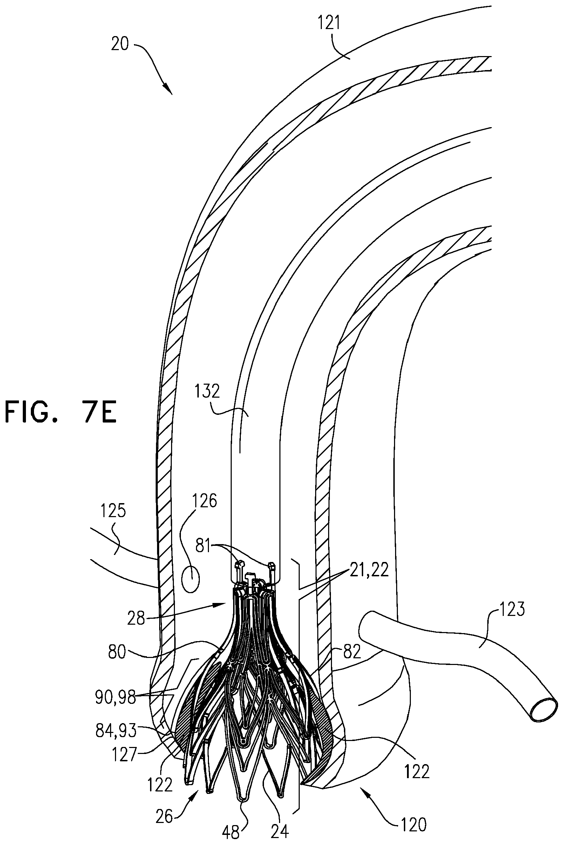

In Fig. 7E, a proximal portion of inner frame 24 is expanded. The proximal portion of inner frame 24 defines an upstream flange. In order to expand inner frame 24, the capsule surrounding inner frame 24 is pushed from around inner frame 24 typically in the proximal direction and then retracted distally through prosthetic valve 22 and finally through aorta 121 and out of the body of the patient. At this stage, the proximal portion of inner frame 24 exerts radial forces against native tissue of the native aortic valve 120 thereby preventing and eliminating migration of prosthetic valve 22 in both the upstream and downstream directions. Additionally, at this stage, the native leaflets 122 are sandwiched between (1) tissue anchors 98 of prosthetic valve 22 and (2) the proximal portion of inner frame 24 at anchoring region 90.

Fig. 7F shows prosthetic valve 22 fully implanted at native aortic valve 120. The distal end 28 of prosthetic valve 22 is allowed to expand by fully retracting delivery tube 132 (not shown) such that tube 132 is disposed distally to and downstream of appendages 81. Once tube 132 does not surround appendages 81, distal end 28 of valve 22 is allowed to expand. Fig. 7F shows the appropriate alignment and sealing of prosthetic valve 22 with native aortic valve 120. As shown, due to the overall height H1 of prosthetic valve 22, distal end 28 of prosthetic valve 22 does not extend downstream toward the aortic sinuses or to the openings 124 and 126 of coronary arteries 123 and 125. In such a manner, due to the overall height of prosthetic valve 22, prosthetic valve 22 does not occlude any of the openings 124 or 126 of coronary arteries 123 and 125. As described hereinabove, height H1 of prosthetic valve 22 is 20-26 mm, e.g., 22.0-24.83 mm. H1 typically does not include appendages 81.

Prosthetic valve 22 is covered by a covering 130 comprising one or more sheets, e.g., fabric, a polyester such a Dacron(TM), or any other suitable material. It is to be noted that prosthetic valve 22 is shown only partially covered by covering 130 for clarity of illustration.

Supra-annular portion 92 is disposed downstream annular plane AP and subannular portion 70 is disposed upstream of annular plane AP. Typically, as described hereinabove, subannular portion 70 is covered by a covering 150, e.g., a fabric, a polyester such a Dacron(TM), velour cloth, an inflatable cuff that is inflatable either with blood or fluid, and/or any other suitable material. By exerting radial forces on the native tissue, subannular portion 70 helps ensure prosthetic valve 22 is sufficiently anchored and sealed with respect to native aortic valve 120 and prevents and eliminates paravalvular leaks.

Anchoring region 90 provides mechanical anchoring and prevents migration of prosthetic valve 22 upstream into the left ventricle, and subannular portion 70 exerts sufficient radial forces against tissue of aortic valve 120 in order to ensure that prosthetic valve 22 remains securely implanted and doesn't migrate in either the upstream or downstream direction. The overall configuration of frame assembly 21 of prosthetic valve 22 ensures that prosthetic valve 22 withstands and endures the high-pressure environment at the native aortic valve 120.

As shown, prosthetic valve 22 is robust in metal struts at anchoring region 90 so as to provide reinforcement of prosthetic valve 22. In particular, (1) reinforcing struts 56a and 56b at reinforced zones 100 of prosthetic valve 22 that defines a semi-double-strutted region of prosthetic valve 22, (2) intermediate struts 40, (3) intermediate nodes 30, (4) junctions 58, (5) intermediate junctions 42, and (6) junctions 84 and 94 of outer frame 80 provide increased structural support to anchoring region 90.

Additionally, the semi-double-strutted region of inner frame 24 enhances the strength of inner frame 24 such that it does not deform in response to hydrodynamic forces and other forces acted upon prosthetic valve 22 by the native tissue.

Additionally, prosthetic valve 22 is robust in metal struts at subannular portion 70 so as to provide reinforcement of prosthetic valve 22. In particular, (1) struts 52, (2) junctions 54, (3) junctions 48a, and (6) junctions 48b provide increased structural support to subannular portion 70.

Overall, the double strut configuration of portions of inner frame 24 and the entire outer frame 80 provide increased structural support to prosthetic valve 22.

Reference is now made to Fig. 8, which is a schematic illustration of an inner frame 224, in accordance with an application of the present invention. For clarity of illustration, Fig. 8 shows a circumferential half of inner frame 224. Inner frame 224 may be assembled with any of the outer frames described herein to form a prosthetic valve frame assembly. By way of example and not limitation, in Fig. 10, described hereinbelow, inner frame 224 is shown assembled with an outer frame 280 to form a prosthetic valve frame assembly 221. Other than as described below, inner frame 224 may be identical to inner frame 24, described hereinabove with reference to Figs. 1A-7F, and may implement any of the features thereof, *mutatis mutandis;* like reference numeral refer to like parts.

Inner frame 224 is shaped so as to define a plurality of intermediate nodes 230 that are intermediate the proximal and distal ends 26 and 28 of inner frame 224. In the configuration shown in Fig. 8 (and Figs. 10, 11A-C, and 12, described hereinbelow), each intermediate node 230 has at least eight struts extending therefrom, e.g., exactly eight struts, as shown.

The struts extending from each intermediate node 230 include:
- distally-extending pair of two struts 44 of the at least eight struts, described hereinabove with reference to Figs. 1A-D, which, in the configuration of Fig. 8, is a first distally-extending pair of two struts 44,
- a second distally-extending pair of two struts 247 of the at least eight struts,
- first proximally-extending pair of two struts 46 of the at least eight struts, described hereinabove with reference to Figs. 1A-D, and
- second proximally-extending pair of two struts 50 of the at least eight struts, described hereinabove with reference to Figs. 1A-D.

For each pair of circumferentially adjacent intermediate nodes 230, for example:
- (i) first distally-extending strut 44a that extends from one intermediate node 230a of the pair of intermediate nodes 230 converges at distal junction 45 with (ii) second distally-extending strut 44b that extends from the other intermediate node 230b of the pair of intermediate nodes 230,
- (i) a third distally-extending strut 247a that extends from the one intermediate node 230a of the pair of intermediate nodes 230 converges at distal junction 45 with (ii) a fourth distally-extending strut 247b that extends from the other intermediate node 230b of the pair of intermediate nodes 230; the third distally-extending strut 247a is distal to the first distally-extending strut 44a; the fourth distally-extending strut 247b is distal to the second distally-extending strut 44b,
- (i) first proximally-extending strut 46a that extends from the one intermediate node 230a of the pair of intermediate nodes 230 converges at proximal junction 48a with (ii) second proximally-extending strut 46b that extends from the other intermediate node 230b of the pair of intermediate nodes 230, and
- (i) third proximally-extending strut 50a that extends from the one intermediate node 230a of the pair of intermediate nodes 230 converges at second proximal junction 48b with (ii) fourth proximally-extending strut 50b that extends from the other intermediate node 230b of the pair of intermediate nodes 230; the third proximally-extending strut 50a is proximal to the first proximally-extending strut 46a; the fourth proximally-extending strut 50b is proximal to the second proximally-extending strut 46b; the second proximal junction 48b is proximal to the first proximal junction 48a; and the first and second proximal junctions 48a and 48b are circumferentially aligned.

For some applications, (i) first intermediate strut 40a extends proximally from intermediate location 39a along first distally-extending strut 44a, and (ii) second intermediate strut 40b extends proximally from intermediate location 39b of second distally-extending strut 44b; intermediate location 39a along first distally-extending strut 44a is between the one intermediate node 230a and distal junction 45; and intermediate location 39b along second distally-extending strut 44b is between the other intermediate node 230b and the distal junction 45. For some of these applications, the first intermediate strut 40a and second intermediate strut 40b have some or all of the characteristics described hereinabove with reference to Figs. 1A-D.

The first distally-extending pair of two struts 44 and the second distally-extending pair of two struts 247 can be described as arranged in a double-strut formation in which the first and the second pairs are arranged alongside each other. The struts of this double-strut formation are arranged in an undulating or zigzag pattern as a series of ascending and descending struts between circumferentially-adjacent distal junctions 45. It is hypothesized by the inventors that the double-strut formation provides radial strength and rigidity to prosthetic valve 22. Additionally, this double-strut formation of inner frame 224 enhances the strength of inner frame 224 such that it does not deform in response to hydrodynamic forces and other forces acted upon prosthetic valve 22 by the native tissue.

For some applications, respective sequentional portions of first and second intermediate struts 40a and 40b extend from respective upstream sides of first and second distally-extending struts 44a and 44b and curve with the angle and directions of curvature described hereinabove with reference to Figs. 1A-B. In addition to providing one or both of the benefits described above with reference to Figs. 1A-B, this shape of first and second intermediate struts 40a and 40b may help maintain the correct distance between the double struts (i.e., first intermediate struts 40 and second distally-extending pair of two struts 247).

As described hereinabove with reference to Figs. 1A-D, 2A-C, and 3A-F regarding inner frame 24, the struts of the distal, outflow portion of inner frame 224 can be described as arranged in a double-strut formation in which pairs of two inner-frame distal struts (distal-end-node-proximally-extending pair of two struts 62 and second distal-end-node-proximally-extending pair of two struts 66) are arranged alongside each other. Also as described hereinabove with reference to Figs. 1A-D, 2A-C, and 3A-F regarding inner frame 24:
- first distal-end-node-proximally-extending strut 62a that extends from one distal end node 32a of the pair of distal end nodes 32 converges at first outflow junction 64a with (ii) second distal-end-node-proximally-extending strut 62b that extends from the other distal end node 32b of the pair of distal end nodes 32,
- third distal-end-node-proximally-extending strut 66a that extends from the one distal end node 32a of the pair of distal end nodes 32 converges at second outflow junction 64b with (ii) third distal-end-node-proximally-extending strut 66b that extends from the other distal end node 32b of the pair of distal end nodes, and
- first and second outflow junctions 64a and 64b are circumferentially aligned.

In the configuration of inner frame 224 shown in Fig. 8, first and second outflow junctions 64a and 64b are joined at an outflow node 264. Optionally, inner frame 24, described hereinabove with reference to Figs. 1A-D, 2A-C, and 3A-F, may implement this feature.

As mentioned above regarding inner frame 24, inner frame 224 is also shaped so as to define the plurality of distal end nodes 32 that are at distal end 28 of inner frame 224. For some applications, each of distal end nodes 32 is shaped so as to define two notches 229 to facilitate stitching of inner frame 224 to outer frame 280.

Reference is now made to Fig. 9, which is a schematic illustration of outer frame 280, in accordance with an application of the present invention. For clarity of illustration, Fig. 9 shows a circumferential half of outer frame 280. Outer frame 280 may be assembled with any of the inner frames described herein to form a prosthetic valve frame assembly. By way of example and not limitation, in Fig. 10, described hereinbelow, outer frame 280 is shown assembled with inner frame 224 to form prosthetic valve frame assembly 221. Other than as described below, outer frame 280 may be identical to outer frame 80, described hereinabove with reference to Figs. 1A-7F, and may implement any of the features thereof, *mutatis mutandis;* like reference numeral refer to like parts.

Outer frame 280 is shaped so as to define a plurality of intermediate nodes 286 that are intermediate the proximal and distal ends 26 and 28 of outer frame 280. In the configuration shown in Fig. 9 (and Fig. 10, described hereinbelow), each intermediate node 286 has at least 12 struts extending therefrom, e.g., exactly 12 struts, as shown.

The struts extending from each intermediate node include:
- first distally-extending pair of two struts 87 (87a and 87b) of the at least 12 struts, described hereinabove with reference to Figs. 2A-C,
- second distally-extending pair of two struts 89 of the at least 12 struts, described hereinabove with reference to Figs. 2A-C,
- a third distally-extending pair of two struts 295 of the at least 12 struts,
- first proximally-extending pair of two struts 85 of the at least 12 struts, described hereinabove with reference to Figs. 2A-C,
- second proximally-extending pair of two struts 83 of the at least 12 struts, described hereinabove with reference to Figs. 2A-C, and
- a third proximally-extending pair of two struts 297 of the at least 12 struts.

For each pair of circumferentially adjacent intermediate nodes 286, for example:
- (i) first distally-extending distal strut 87a that extends from one intermediate node 286a of the pair of intermediate nodes 286 converges at distal end node 88 with (ii) second distally-extending distal strut 87b that extends from the other intermediate node 286b of the pair of intermediate nodes 286,
- (i) third distally-extending distal strut 89a that extends from one intermediate node 286a of the pair of intermediate nodes 286 converges at distal end node 88 with (ii) fourth distally-extending distal strut 89b that extends from the other intermediate node 286b of the pair of intermediate nodes 286,
- (i) a fifth distally-extending strut 295a that extends from one intermediate node 286a of the pair of intermediate nodes 286 converges at a distal intermediate node 296 with (ii) a sixth distally-extending strut 295b that extends from the other intermediate node 286b of the pair of intermediate nodes 286,
- (i) first proximally-extending proximal strut 85a that extends from the one intermediate node 286a of the pair of intermediate nodes 286 converges at first outer-frame proximal junction 94 with (ii) second proximally-extending proximal strut 85b that extends from the other intermediate node 286b of the pair of intermediate nodes 286,
- (i) third proximally-extending proximal strut 83a that extends from the one intermediate node 286a of the pair of intermediate nodes 286 converges at second outer-frame proximal junction 84 with (ii) fourth proximally-extending proximal strut 83b that extends from the other intermediate node 286b of the pair of intermediate nodes 286, and
- (i) a fifth proximally-extending strut 297a that extends from one intermediate node 286a of the pair of intermediate nodes 286 converges at a proximal intermediate node 299 with (ii) a sixth proximally-extending strut 297b that extends from the other intermediate node 286b of the pair of intermediate nodes 286.

Optionally, distal intermediate node 296 and proximal intermediate node 299 are circumferentially aligned, such as shown.

The first distally-extending pair of two struts 87, the second distally-extending pair of two struts 89, and the third distally-extending pair of two struts 295 can be described as arranged in multiple-strut formation, such as a triple-strut formation, in which the first, the second, and the third pairs are arranged alongside each other. Optionally, (i) an average distance between the second distally-extending pair of two struts 89 and the third distally-extending pair of two struts 295 is greater than (ii) an average distance between the first distally-extending pair of two struts 87 and the second distally-extending pair of two struts 89, such as shown in Figs. 9 and 10.

Optionally, intermediate nodes 286 are shaped to as to define respective narrow waists, which may serve as stitching point between the inner and the outer frames, such as shown.

As mentioned above regarding outer frame 80, outer frame 280 is also shaped so as to define the plurality of distal end nodes 88 that are at distal end 28 of outer frame 280. For some applications, each of distal end nodes 88 is shaped so as to define two notches 233 to facilitate stitching of outer frame 280 to inner frame 224.

Reference is now made to Fig. 10, which is a schematic illustrations of a prosthetic valve 222 comprising a prosthetic valve frame assembly 221 comprising inner frame 224 and outer frame 280, in accordance with some applications of the invention. For clarity of illustration, Fig. 10 shows a circumferential half of prosthetic valve 222. Also for clarity of illustration, Fig. 10 omits prosthetic leaflets 140, although prosthetic valve 222 comprises prosthetic leaflets 140. Optionally, prosthetic valve 222 further comprises skirt 300, such as described hereinbelow with reference to Figs. 11A-C.

Reference is now made to Figs. 11A-B, which are schematic illustrations of inner frame 224 of prosthetic valve 222, including skirt 300 and prosthetic leaflets 140, in accordance with respective applications of the present invention.

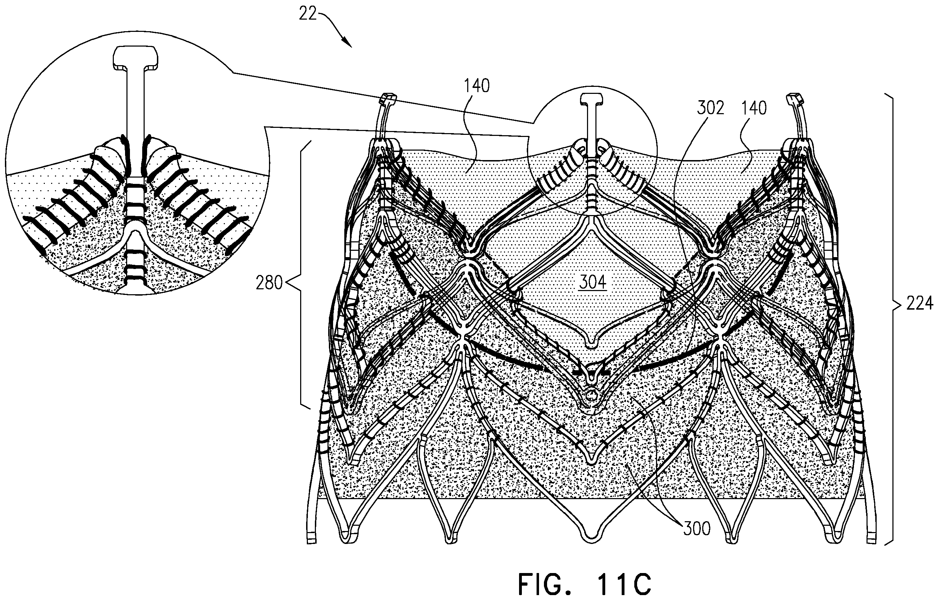

Reference is also made to Fig. 11C, which is a schematic illustration of prosthetic valve frame assembly 221 of prosthetic valve 222, including skirt 300 and prosthetic leaflets 140, in accordance with an application of the present invention.

The features of Figs. 11A-B and/or 11C may be incorporated into any of the other prosthetic valves described herein.

In Figs. 11A-C, prosthetic leaflets 140 are partially visible and partially concealed by skirt 300. Prosthetic leaflets 140 can perhaps better be seen in Figs. 3E-F, which show frame assembly 21 of prosthetic valve 22. The stitching that couples prosthetic leaflets 140 to skirt 300 can be seen in Figs. 11A-C, as described below.

For some applications, prosthetic valve 222 comprises skirt 300, which covers a portion of inner frame 224 of prosthetic valve 222, such as shown. Skirt 300 may help prevent paravalvular leak (PVL). Skirt 300 may be attached to an inner surface of inner frame 224 (such as shown) and/or to an external surface of the inner frame (configuration not shown). Alternatively, skirt 300 may be provided covering a portion of outer frame 280 (configuration not shown). For example, skirt 300 may comprise polyethylene terephthalate (PET), polyurethane (PU), or pericardium, such as bovine or porcine pericardium.

In configurations in which skirt 300 covers a portion of prosthetic valve 222 at the height of prosthetic leaflets 140 (such as shown), skirts 300 may be shaped so as to define openings 304, which may provide coronary access, such as for future percutaneous coronary intervention (PCI).

For some applications, prosthetic leaflets 140 are coupled to skirt 300, such as by stitching 302, which may, for example, comprise polyester, e.g., braided polyester.

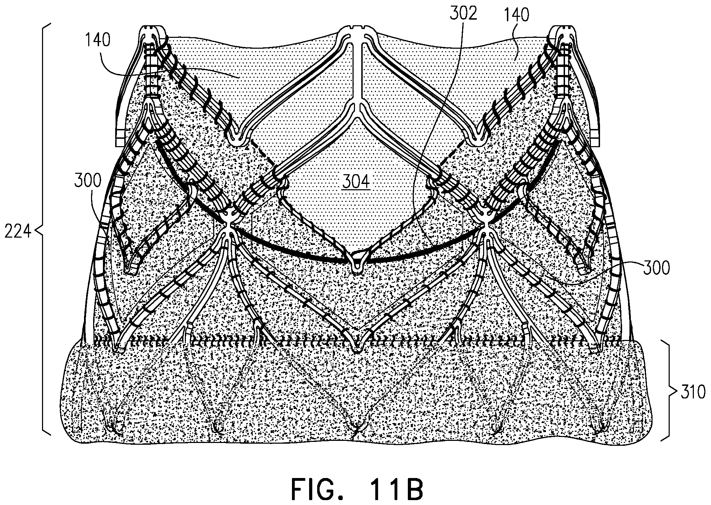

For some applications, prosthetic valve 222 comprises an upstream sealing cuff 310, in addition to or instead of skirt 300, such as shown in Fig. 11B. Upstream sealing cuff 310 may help prevent paravalvular leak (PVL). Upstream sealing cuff 310 may optionally be folded over upstream proximal end 26 of prosthetic valve 222, such as shown. Upstream sealing cuff 310 may be coupled to an upstream end of portion of skirt 300, such as by stitching, or may be integral with skirt 300, i.e., fabricated from the same piece(s) of material as the skirt. Upstream sealing cuff 310 may be attached to inner frame 224 (such as shown) and/or to outer frame 280 (configuration not shown). For example, upstream sealing cuff 310 may comprise polyethylene terephthalate (PET), polyurethane (PU), or pericardium, such as bovine or porcine pericardium. Alternatively, prosthetic valve 222 does not comprise upstream sealing cuff 310, such as shown in Figs. 11A and 11C.

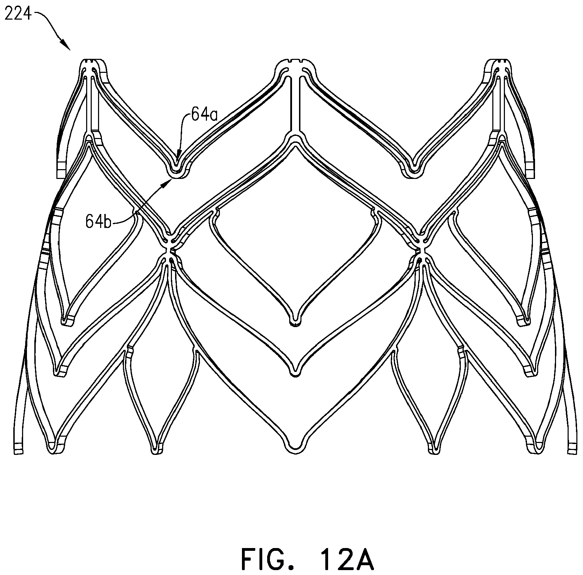

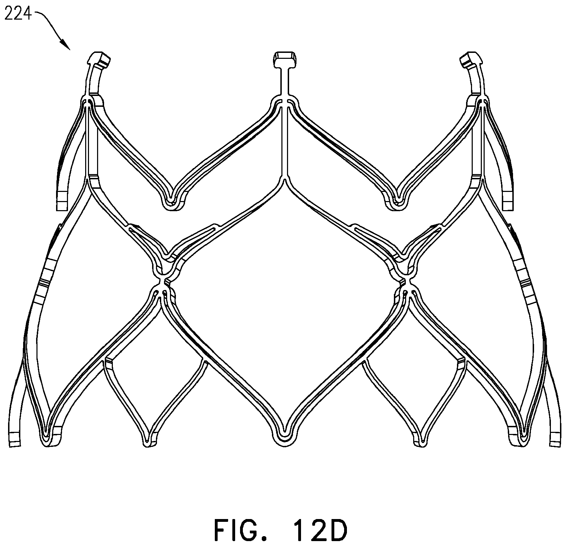

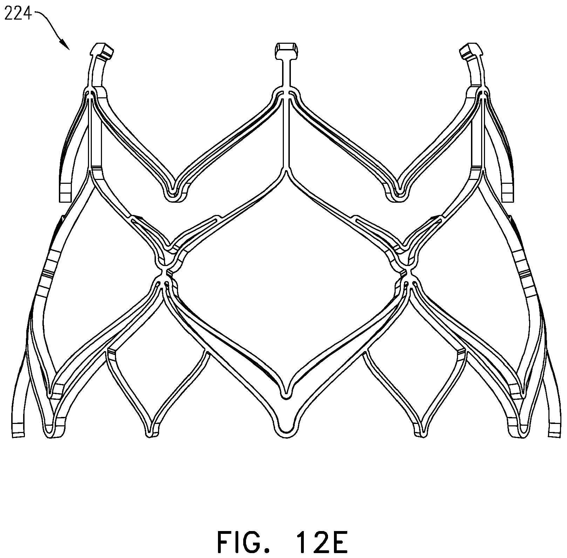

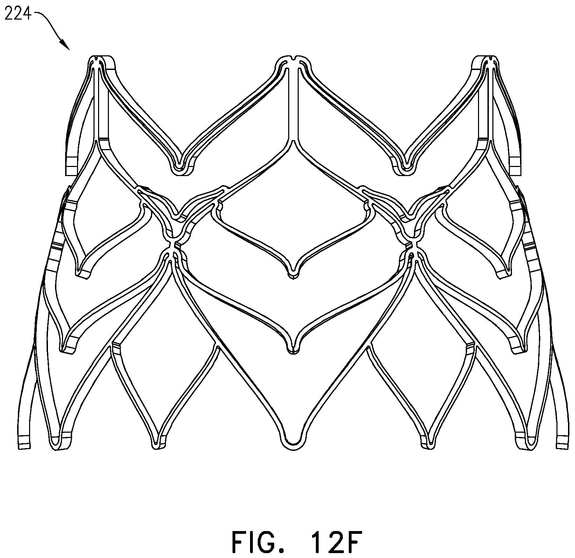

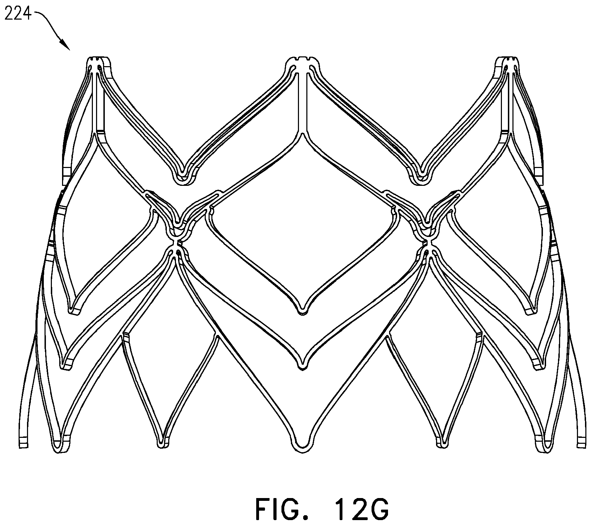

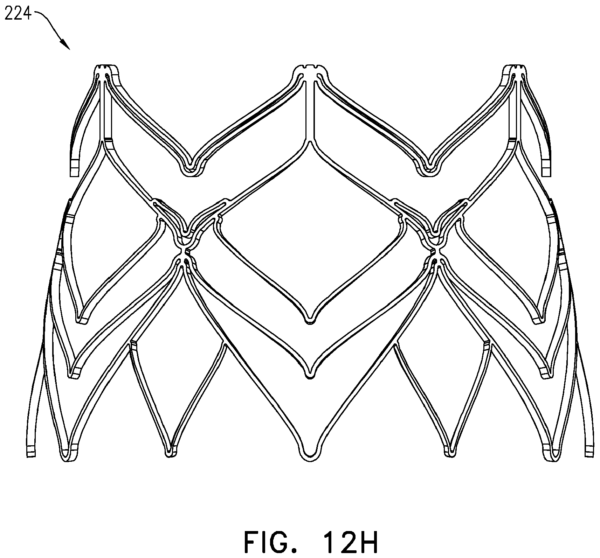

Reference is now made to Figs. 12A-H, which are schematic illustrations of additional configurations of inner frame 224, in accordance with respective applications of the present invention. For clarity of illustration, Figs. 12A-H show circumferential halves of inner frame 224. These configurations may be implemented in combination with, or instead of, any of the configurations of the inner frame described herein.

The configuration of inner frame 224 shown in Fig. 12A is identical to the configuration of inner frame 224 shown in Fig. 8, except that first and second outflow junctions 64a and 64b are not joined at outflow node 264. In this respect, the configuration of inner frame 224 shown in Fig. 12A is similar to the configuration of inner frame 24 shown in Figs. 1A-C.

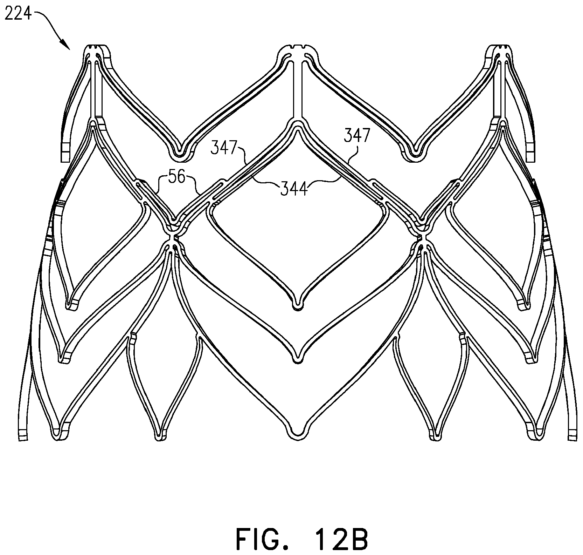

In the configuration of inner frame 224 shown in Fig. 12B, inner frame 224 is shaped so as to define:
- reinforcing struts 56, such as described hereinabove with reference to Figs. 1A-C,
- a first distally-extending pair of two struts 344, similar to the first distally-extending pair of two struts 44, described hereinabove with reference to Figs. 1A-C, and
- a second distally-extending pair of two struts 347, similar to the second distally-extending pair of two struts 247 described hereinabove with reference to Fig. 10.

Collectively, these struts can be described as arranged in a partial triple-strut formation and partial a double-strut formation.

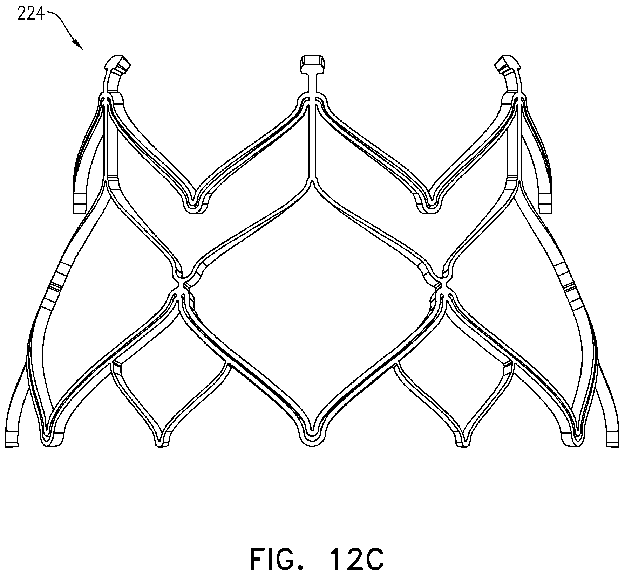

Figs. 12C-H show additional alternative configurations of inner frame 224.

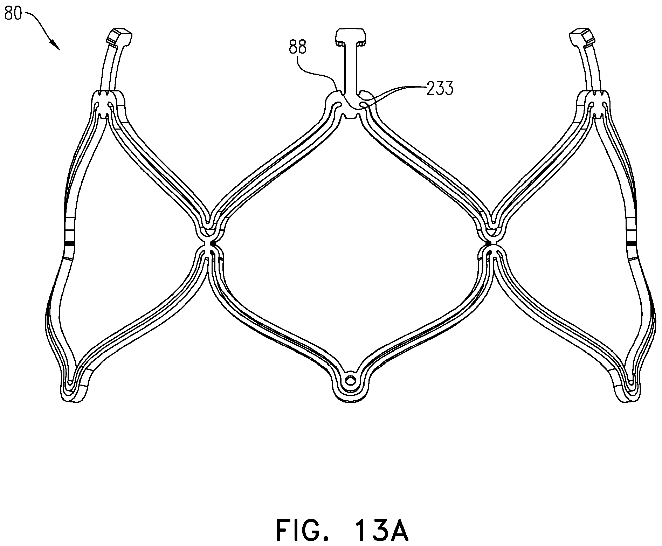

Reference is now made to Figs. 13A-C, which are schematic illustrations of additional configurations of outer frame 80, in accordance with respective applications of the present invention. For clarity of illustration, Figs. 13A-C show circumferential halves of outer frame 80.

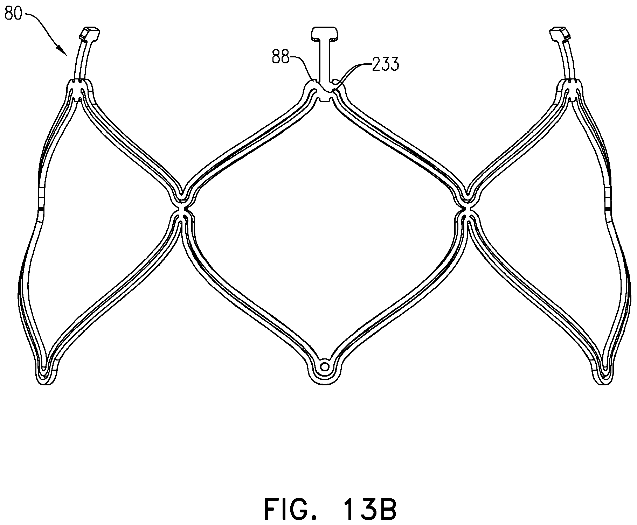

The configurations of outer frame 80 shown in Figs. 13B-C are generally similar to the configuration shown in Fig. 2A, except that each of distal end nodes 88 is shaped so as to define two notches 233 to facilitate stitching of the outer frame to the inner frame, such as described hereinabove with reference to Fig. 9 for outer frame 280. The shape of the struts is somewhat different in the configuration shown in Fig. 13B from the configuration shown in Fig. 13C. Either of these shapes may be implemented in any of the configurations of outer frames described herein, *mutatis mutandis.*

Reference is now made to Fig. 14, which is a schematic illustration of an outer frame 380, in accordance with an application of the present invention. For clarity of illustration, Fig. 14 shows a circumferential half of outer frame 380. The features of this configuration may be implemented in combination with, or instead of, any of the other outer frames described herein. Other than as described below, this configuration is generally similar to the configuration of outer frame 80 described hereinabove with reference to Figs. 2A-C.

In outer frame 380, each intermediate node 86 has at least eight struts extending therefrom, such as shown, and the struts extending from each intermediate node 86 include (a) a first distally-extending pair of two struts 387 (387a and 387b) of the at least eight struts, (b) second distally-extending pair of two struts 89 of the at least eight struts, (c) a first proximally-extending pair of two struts 385 of the at least eight struts, and (d) second proximally-extending pair of two struts 83 of the at least eight struts. These four pairs of struts together define a stent cell 390.

First proximally-extending pair of two struts 385 includes (i) a first proximally-extending proximal strut 385a that extends from the one intermediate node 86a of the pair of intermediate nodes 86 and converges at first outer-frame proximal junction 94 with (ii) a second proximally-extending proximal strut 385b that extends from the other intermediate node 86b of the pair of intermediate nodes 86

First distally-extending pair of two struts 387 includes (i) a first distally-extending distal strut 387a that extends from one intermediate node 86a of the pair of intermediate nodes 86 and converges at distal end node 88 with (ii) a second distally-extending distal strut 387b that extends from the other intermediate node 86b of the pair of intermediate nodes 86.

Outer frame 380 is further shaped so as to define, for each stent cell 390 defined as described above, four struts arranged in a generally X-shaped arrangement within stent cell 390 as follows:
- first and second distal struts 392a and 392b extend from first distally-extending distal strut 387a and second distally-extending distal strut 387b, respectively, and converge at a central junction 396, and
- first and second proximal struts 394a and 394b extend from first proximally-extending proximal strut 385a and second proximally-extending proximal strut 385b, respectively, and converge at central junction 396.

Reference is now made to Figs. 1A-7F. It is to be noted that although the prosthetic valves described in are described as being configured for implantation at a native aortic valve of the patient, the prosthetic valves may also be configured, *mutatis mutandis,* to be implanted at other cardiac valves of the patient, e.g., the pulmonary valve, the mitral valve and the tricuspid valve.

## Claims

1. A prosthetic heart valve (22, 222) configured for transcatheter implantation, the prosthetic heart valve (22, 222) comprising:
a prosthetic valve frame that surrounds a central longitudinal axis of the prosthetic heart valve (22, 222) to define a lumen along the axis when the prosthetic heart valve (22, 222) is in an expanded state; and
a plurality of prosthetic leaflets (140), disposed within the lumen, and arranged to facilitate one-way inflow-to-outflow fluid flow through the lumen in a proximal-to-distal direction when the prosthetic heart valve (22, 222) is in the expanded state,
wherein the prosthetic valve frame:
comprises a plurality of struts, and
is shaped so as to define a plurality of intermediate nodes (30, 230) that are intermediate proximal and distal ends (26, 28) of the prosthetic valve frame, wherein a portion of the plurality of struts are intermediate struts (40),
wherein each intermediate node (30, 230) has at least four struts extending therefrom, the at least four struts comprising (a) a distally-extending pair of two struts (44) of the at least four struts, and (b) a proximally-extending pair of two struts (46) of the at least four struts,
wherein for each pair of circumferentially adjacent intermediate nodes (30, 230):
(i) a first distally-extending strut (44a) that extends from one intermediate node (30a, 230a) of the pair of intermediate nodes (30, 230) converges at a distal junction (45) with (ii) a second distally-extending strut (44b) that extends from the other intermediate node (30b, 230b) of the pair of intermediate nodes (30, 230),
(i) a first proximally-extending strut (46a) that extends from the one intermediate node (30a, 230a) of the pair of intermediate nodes (30, 230) converges at a proximal junction (48a) with (ii) a second proximally-extending strut (46b) that extends from the other intermediate node (30b, 230b) of the pair of intermediate nodes (30, 230), and
(i) a first intermediate strut (40a) extends proximally from an intermediate location (39a) along the first distally-extending strut (44a), and (ii) a second intermediate strut (40b) extends proximally from an intermediate location (39b) along the second distally-extending strut (44b), the intermediate location (39a) along the first distally-extending strut (44a) being between the one intermediate node (30a, 230a) and the distal junction (45), and the intermediate location (39b) along the second distally-extending strut (44b) being between the other intermediate node (30b, 230b) and the distal junction (45),
wherein the first and the second intermediate struts (40a, 40b) converge at an intermediate junction (42) that points in a proximal direction,
wherein the intermediate junction (42) is intermediate the distal junction (45) and the proximal junction (48a), and
wherein the proximal junction (48a), the intermediate junction (42), and the distal junction (45) are circumferentially aligned, **characterised in that**:
each intermediate node (30, 230) has at least six struts extending therefrom,
the proximally-extending pair of the two struts (46) defines a first proximally-extending pair of two struts (46) of the at least six struts,
the at least six struts comprise a second proximally-extending pair of two struts (50) of the at least six struts,
the proximal junction (48a) defines a first proximal junction (48a), and
for each pair of circumferentially adjacent intermediate nodes (30, 230):
(i) a third proximally-extending strut (50a) that extends from the one intermediate node (30a, 230a) of the pair of intermediate nodes (30, 230) converges at a second proximal junction (48b) with (ii) a fourth proximally-extending strut (50b) that extends from the other intermediate node (30b, 230b) of the pair of intermediate nodes (30, 230),
the third proximally-extending strut (50a) is proximal to the first proximally-extending strut (46a),
the fourth proximally-extending strut (50b) is proximal to the second proximally-extending strut (46b),
the second proximal junction (48b) is proximal to the first proximal junction (48a), and
the second proximal junction (48b) and the first proximal junction (48a) are circumferentially aligned.

2. The prosthetic heart valve according to claim 1, wherein when the prosthetic heart valve (22, 222) is in the expanded state, for each pair of circumferentially adjacent intermediate nodes (30, 230):
a smaller rhombus (10) of the prosthetic valve frame is bounded by (1) distal portions of the first and the second distally-extending struts (44a, 44b), and (2) the first and the second intermediate struts (40a, 40b), and
a larger rhombus (12) of the prosthetic valve frame is bounded by (1) the first and the second distally-extending struts (44a, 44b), and (2) the first and the second proximally-extending struts (46a, 46b),
wherein the smaller rhombus (10) is within the larger rhombus (12).

3. The prosthetic heart valve according to claim 1, wherein the first and the second intermediate struts (40a, 40b) have respective sequential longitudinal portions, and wherein when the prosthetic heart valve (22, 222) is in the expanded state, the respective sequential longitudinal portions:
extend at an angle of 75 - 105 degrees from respective upstream sides of the first and the second distally-extending struts (44a, 44b), respectively,
curve partially upstream and partially toward the respective closest intermediate nodes (30a, 230a, 30b, 230b) along respective first curved longitudinal portions (41) of the first and the second intermediate struts (40a, 40b), and
curve partially upstream and partially away from the respective closest intermediate nodes (30a, 230a, 30b, 230b) along respective second curved longitudinal portions (43) of the first and the second intermediate struts (40a, 40b).

4. The prosthetic heart valve according to claim 3, wherein each of the first curved longitudinal portions (41) has a length of 0.2 - 0.75 mm, and each of the second curved longitudinal portions (43) has a length of 1 - 1.75 mm, the lengths measured along respective curved central longitudinal axes of the curved longitudinal portions (41, 43).

5. The prosthetic heart valve according to claim 3,
wherein each of the first curved longitudinal portions (41) has a first length and each of the second curved longitudinal portions (43) has a second length, the lengths measured along respective curved central longitudinal axes of the curved longitudinal portions (41, 43), and
wherein a ratio of the second length to the first length is 2 - 8.

6. The prosthetic heart valve according to claim 3, wherein each of the first curved longitudinal portions (41) has a first inner radius of curvature of 0.05 - 0.2 mm, and each of the second curved longitudinal portions (43) has a second inner radius of curvature of 0.4 - 0.8 mm.

7. The prosthetic heart valve according to claim 3,
wherein each of the first curved longitudinal portions (41) has a first inner radius of curvature and each of the second curved longitudinal portions (43) has a second inner radius of curvature, and
wherein a ratio of the second inner radius of curvature to the first inner radius of curvature is 2 - 10.

8. The prosthetic heart valve according to claim 1, wherein the prosthetic valve frame has an overall height of 20-26 mm when the prosthetic heart valve (22, 222) is in the expanded state.

9. The prosthetic heart valve according to any one of claims 1-8, wherein when the prosthetic heart valve (22, 222) is in the expanded state, for each pair of circumferentially adjacent intermediate nodes (30, 230):
a smaller rhombus (10) of the prosthetic valve frame is bounded by (1) distal portions of the first and the second distally-extending struts (44a, 44b), and (2) the first and second intermediate struts (40a, 40b),
a larger rhombus (12) of the prosthetic valve frame is bounded by (1) the first and the second distally-extending struts (44a, 44b), and (2) the first and the second proximally-extending struts (46a, 46b), and
a largest rhombus (14) of the prosthetic valve frame is bounded by (1) the first and the second distally-extending struts (44a, 44b), and (2) the third and the fourth proximally-extending struts (50a, 50b),
wherein the smaller rhombus (10) is within the larger rhombus (12), and the larger rhombus (12) is within the largest rhombus (14).

10. The prosthetic heart valve according to any one of claims 1-8,
wherein the portion of the plurality of struts comprises a first portion of the plurality of struts,
wherein the prosthetic valve frame comprises a second portion of the plurality of struts,
wherein the second portion of the plurality of struts are proximal struts (52), and
wherein for each intermediate node (30, 230):
(i) a first proximal strut (52a) extends proximally from an intermediate location (53a) along a first strut (50') of the second proximally-extending pair of the two struts (50) of the at least six struts, and (ii) a second proximal strut (52b) extends proximally from an intermediate location (53b) along a second strut (50") of the second proximally-extending pair of the two struts (50) of the at least six struts, each intermediate location (53a, 53b) along the respective first and second struts (50', 50") of the second proximally-extending pair of two struts (50) being between the intermediate node (30, 230) and respective proximal ends of each of the first and the second struts (50', 50") of the second proximally-extending pair of two struts (50),
the first and the second proximal struts (52a, 52b) converge at a proximally-pointing junction (54) of the first and the second proximal struts (52a, 52b),
the proximally-pointing junction (54) of the first and second proximal struts (52a, 52b) is circumferentially aligned with the intermediate node (30, 230), and the proximally-pointing junction (54) is circumferentially offset with respect to the second proximal junction (48b).

11. The prosthetic heart valve according to claim 10, wherein the proximally-pointing junctions (54) are longitudinally aligned with the second proximal junctions (48b) at the proximal end (26) of the prosthetic valve frame.

12. The prosthetic heart valve (222) according to any one of claims 1-8,
wherein each intermediate node (230) has at least eight struts extending therefrom,
wherein the distally-extending pair of two struts (44) is a first distally-extending pair of two struts (44) of the at least eight struts, and
wherein for each pair of circumferentially adjacent intermediate nodes (230):
(i) a third distally-extending strut (247a) that extends from the one intermediate node (230a) of the pair of intermediate nodes (230) converges at the distal junction (45) with (ii) a fourth distally-extending strut (247b) that extends from the other intermediate node (230b) of the pair of intermediate nodes (230),
the third distally-extending strut (247a) is distal to the first distally-extending strut (44a), and
the fourth distally-extending strut (247b) is distal to the second distally-extending strut (44b).

13. The prosthetic heart valve according to any one of claims 1-12,
wherein the prosthetic valve frame is an inner frame (24, 224) that surrounds the central longitudinal axis of the prosthetic heart valve (22, 222) to define the lumen along the axis when the prosthetic heart valve (22, 222) is in the expanded state, and
wherein the prosthetic heart valve (22, 222) comprises a prosthetic valve frame assembly (21) comprising the inner frame (24, 224) and an outer frame (80, 280, 380) that is coupled to and surrounds the inner frame (24, 224).

## Patentansprüche

1. Herzklappenprothese (22, 222), die für eine Transkatheterimplantation konfiguriert ist, wobei die Herzklappenprothese (22, 222) Folgendes umfasst:
einen Klappenprothesenrahmen, der eine Mittellängsachse der Herzklappenprothese (22, 222) umgibt, um ein Lumen entlang der Achse zu definieren, wenn sich die Herzklappenprothese (22, 222) in einem ausgedehnten Zustand befindet; und
eine Vielzahl von Segelprothesen (140), die innerhalb des Lumens positioniert und so angeordnet sind, dass sie einen Einweg-Zufluss-Abfluss-Fluidstrom durch das Lumen in einer proximal-distalen Richtung ermöglichen, wenn sich die Herzklappenprothese (22, 222) in dem ausgedehnten Zustand befindet,
wobei der Klappenprothesenrahmen:
eine Vielzahl von Streben umfasst und
so geformt ist, dass er eine Vielzahl von Zwischenknoten (30, 230) definiert, die zwischen dem proximalen und dem distalen Ende (26, 28) des Klappenprothesenrahmens liegen,
wobei ein Teil der Vielzahl von Streben Zwischenstreben (40) sind,
wobei jeder Zwischenknoten (30, 230) mindestens vier Streben aufweist, die sich davon erstrecken, wobei die mindestens vier Streben (a) ein sich distal erstreckendes Paar von zwei Streben (44) der mindestens vier Streben und (b) ein sich proximal erstreckendes Paar von zwei Streben (46) der mindestens vier Streben umfassen,
wobei für jedes Paar von in Umfangsrichtung benachbarten Zwischenknoten (30, 230):
(i) eine erste sich distal erstreckende Strebe (44a), die sich von einem Zwischenknoten (30a, 230a) des Paars von Zwischenknoten (30, 230) erstreckt, an einer distalen Verbindungsstelle (45) mit (ii) einer zweiten sich distal erstreckenden Strebe (44b) konvergiert, die sich von dem anderen Zwischenknoten (30b, 230b) des Paars von Zwischenknoten (30, 230) erstreckt,
(i) eine erste sich proximal erstreckende Strebe (46a), die sich von dem einen Zwischenknoten (30a, 230a) des Paars von Zwischenknoten (30, 230) erstreckt, an einer proximalen Verbindungsstelle (48a) mit (ii) einer zweiten sich proximal erstreckenden Strebe (46b) konvergiert, die sich von dem anderen Zwischenknoten (30b, 230b) des Paars von Zwischenknoten (30, 230) erstreckt, und
(i) eine erste Zwischenstrebe (40a) sich proximal von einer Zwischenposition (39a) entlang der ersten sich distal erstreckenden Strebe (44a) erstreckt, und (ii) eine zweite Zwischenstrebe (40b) sich proximal von einer Zwischenposition (39b) entlang der zweiten sich distal erstreckenden Strebe (44b) erstreckt, wobei die Zwischenposition (39a) entlang der ersten sich distal erstreckenden Strebe (44a) zwischen dem einen Zwischenknoten (30a, 230a) und der distalen Verbindungsstelle (45) liegt, und die Zwischenposition (39b) entlang der zweiten sich distal erstreckenden Strebe (44b) zwischen dem anderen Zwischenknoten (30b, 230b) und der distalen Verbindungsstelle (45) liegt,
wobei die erste und die zweite Zwischenstrebe (40a, 40b) an einer Zwischenverbindungsstelle (42) konvergieren, die in eine proximale Richtung zeigt,
wobei die Zwischenverbindungsstelle (42) zwischen der distalen Verbindungsstelle (45) und der proximalen Verbindungsstelle (48a) liegt, und
wobei die proximale Verbindungsstelle (48a), die Zwischenverbindungsstelle (42) und die distale Verbindungsstelle (45) in Umfangsrichtung ausgerichtet sind, **dadurch gekennzeichnet, dass**:
jeder Zwischenknoten (30, 230) mindestens sechs Streben aufweist, die sich davon erstrecken,
das sich proximal erstreckende Paar der zwei Streben (46) ein erstes sich proximal erstreckendes Paar von zwei Streben (46) der mindestens sechs Streben definiert,
die mindestens sechs Streben ein zweites sich proximal erstreckendes Paar von zwei Streben (50) der mindestens sechs Streben umfassen,
die proximale Verbindungsstelle (48a) eine erste proximale Verbindungsstelle (48a) definiert, und
für jedes Paar von in Umfangsrichtung benachbarten Zwischenknoten (30, 230):
(i) eine dritte sich proximal erstreckende Strebe (50a), die sich von dem einen Zwischenknoten (30a, 230a) des Paars von Zwischenknoten (30, 230) erstreckt, an einer zweiten proximalen Verbindungsstelle (48b) mit (ii) einer vierten sich proximal erstreckenden Strebe (50b) konvergiert, die sich von dem anderen Zwischenknoten (30b, 230b) des Paars von Zwischenknoten (30, 230) erstreckt,
die dritte sich proximal erstreckende Strebe (50a) proximal zu der ersten sich proximal erstreckenden Strebe (46a) liegt,
die vierte sich proximal erstreckende Strebe (50b) proximal zu der zweiten sich proximal erstreckenden Strebe (46b) liegt,
die zweite proximale Verbindungsstelle (48b) proximal zu der ersten proximalen Verbindungsstelle (48a) liegt, und
die zweite proximale Verbindungsstelle (48b) und die erste proximale Verbindungsstelle (48a) in Umfangsrichtung ausgerichtet sind.

2. Herzklappenprothese nach Anspruch 1, wobei, wenn sich die Herzklappenprothese (22, 222) in dem ausgedehnten Zustand befindet, für jedes Paar von in Umfangsrichtung benachbarten Zwischenknoten (30, 230):
eine kleinere Raute (10) des Klappenprothesenrahmens durch (1) distale Abschnitte der ersten und der zweiten sich distal erstreckenden Strebe (44a, 44b) und (2) die erste und die zweite Zwischenstrebe (40a, 40b) begrenzt wird, und
eine größere Raute (12) des Klappenprothesenrahmens durch (1) die erste und die zweite sich distal erstreckende Strebe (44a, 44b) und (2) die erste und die zweite sich proximal erstreckende Strebe (46a, 46b) begrenzt wird,
wobei die kleinere Raute (10) innerhalb der größeren Raute (12) liegt.

3. Herzklappenprothese nach Anspruch 1, wobei die erste und die zweite Zwischenstrebe (40a, 40b) jeweilige aufeinanderfolgende Längsabschnitte aufweisen, und wobei, wenn sich die Herzklappenprothese (22, 222) in dem ausgedehnten Zustand befindet, die jeweiligen aufeinanderfolgenden Längsabschnitte:
sich in einem Winkel von 75 - 105 Grad von den jeweiligen stromaufwärts gelegenen Seiten der ersten beziehungsweise der zweiten sich distal erstreckenden Strebe (44a, 44b) erstrecken,
sich teilweise stromaufwärts und teilweise zu den jeweiligen nächstgelegenen Zwischenknoten (30a, 230a, 30b, 230b) hin entlang jeweiliger erster gekrümmter Längsabschnitte (41) der ersten und der zweiten Zwischenstrebe (40a, 40b) krümmen, und
sich teilweise stromaufwärts und teilweise von den jeweiligen nächstgelegenen Zwischenknoten (30a, 230a, 30b, 230b) entlang jeweiliger zweiter gekrümmter Längsabschnitte (43) der ersten und der zweiten Zwischenstrebe (40a, 40b) weg krümmen.

4. Herzklappenprothese nach Anspruch 3, wobei jeder der ersten gekrümmten Längsabschnitte (41) eine Länge von 0,2 - 0,75 mm aufweist und jeder der zweiten gekrümmten Längsabschnitte (43) eine Länge von 1 - 1,75 mm aufweist, wobei die Längen entlang jeweiliger gekrümmter Mittellängsachsen der gekrümmten Längsabschnitte (41, 43) gemessen werden.

5. Herzklappenprothese nach Anspruch 3,
wobei jeder der ersten gekrümmten Längsabschnitte (41) eine erste Länge aufweist und jeder der zweiten gekrümmten Längsabschnitte (43) eine zweite Länge aufweist, wobei die Längen entlang jeweiliger gekrümmter Mittellängsachsen der gekrümmten Längsabschnitte (41, 43) gemessen werden, und
wobei ein Verhältnis der zweiten Länge zu der ersten Länge 2 - 8 beträgt.

6. Herzklappenprothese nach Anspruch 3, wobei jeder der ersten gekrümmten Längsabschnitte (41) einen ersten inneren Krümmungsradius von 0,05 - 0,2 mm aufweist und jeder der zweiten gekrümmten Längsabschnitte (43) einen zweiten inneren Krümmungsradius von 0,4 - 0,8 mm aufweist.

7. Herzklappenprothese nach Anspruch 3,
wobei jeder der ersten gekrümmten Längsabschnitte (41) einen ersten inneren Krümmungsradius aufweist und jeder der zweiten gekrümmten Längsabschnitte (43) einen zweiten inneren Krümmungsradius aufweist, und
wobei ein Verhältnis des zweiten inneren Krümmungsradius zu dem ersten inneren Krümmungsradius 2 - 10 beträgt.

8. Herzklappenprothese nach Anspruch 1, wobei der Klappenprothesenrahmen eine Gesamthöhe von 20-26 mm aufweist, wenn sich die Herzklappenprothese (22, 222) in dem ausgedehnten Zustand befindet.

9. Herzklappenprothese nach einem der Ansprüche 1-8, wobei, wenn sich die Herzklappenprothese (22, 222) in dem ausgedehnten Zustand befindet, für jedes Paar von in Umfangsrichtung benachbarten Zwischenknoten (30, 230):
eine kleinere Raute (10) des Klappenprothesenrahmens durch (1) distale Abschnitte der ersten und der zweiten sich distal erstreckenden Strebe (44a, 44b) und (2) die erste und die zweite Zwischenstrebe (40a, 40b) begrenzt wird,
eine größere Raute (12) des Klappenprothesenrahmens durch (1) die erste und die zweite sich distal erstreckende Strebe (44a, 44b) und (2) die erste und die zweite sich proximal erstreckende Strebe (46a, 46b) begrenzt wird, und
eine größte Raute (14) des Klappenprothesenrahmens durch (1) die erste und die zweite sich distal erstreckende Strebe (44a, 44b) und (2) die dritte und die vierte sich proximal erstreckende Strebe (50a, 50b) begrenzt wird,
wobei die kleinere Raute (10) innerhalb der größeren Raute (12) liegt und die größere Raute (12) innerhalb der größten Raute (14) liegt.

10. Herzklappenprothese nach einem der Ansprüche 1-8,
wobei der Teil der Vielzahl von Streben einen ersten Teil der Vielzahl von Streben umfasst,
wobei der Klappenprothesenrahmen einen zweiten Teil der Vielzahl von Streben umfasst,
wobei der zweite Teil der Vielzahl von Streben proximale Streben (52) sind, und
wobei für jeden Zwischenknoten (30, 230):
(i) eine erste proximale Strebe (52a) sich proximal von einer Zwischenposition (53a) entlang einer ersten Strebe (50') des zweiten sich proximal erstreckenden Paars der zwei Streben (50) der mindestens sechs Streben erstreckt, und (ii) eine zweite proximale Strebe (52b) sich proximal von einer Zwischenposition (53b) entlang einer zweiten Strebe (50") des zweiten sich proximal erstreckenden Paars der zwei Streben (50) der mindestens sechs Streben erstreckt, wobei jede Zwischenposition (53a, 53b) entlang der jeweiligen ersten und zweiten Strebe (50', 50") des zweiten sich proximal erstreckenden Paars von zwei Streben (50) zwischen dem Zwischenknoten (30, 230) und jeweiligen proximalen Enden jeder von der ersten und der zweiten Strebe (50', 50") des zweiten sich proximal erstreckenden Paars von zwei Streben (50) liegt,
die erste und die zweite proximale Strebe (52a, 52b) an einer nach proximal zeigenden Verbindungsstelle (54) der ersten und der zweiten proximalen Strebe (52a, 52b) konvergieren,
die nach proximal zeigende Verbindungsstelle (54) der ersten und der zweiten proximalen Strebe (52a, 52b) in Umfangsrichtung mit dem Zwischenknoten (30, 230) ausgerichtet ist, und die nach proximal zeigende Verbindungsstelle (54) in Bezug auf die zweite proximale Verbindungsstelle (48b) in Umfangsrichtung versetzt ist.

11. Herzklappenprothese nach Anspruch 10, wobei die nach proximal zeigenden Verbindungsstellen (54) in Längsrichtung mit den zweiten proximalen Verbindungsstellen (48b) an dem proximalen Ende (26) des Klappenprothesenrahmens ausgerichtet sind.

12. Herzklappenprothese (222) nach einem der Ansprüche 1-8,
wobei jeder Zwischenknoten (230) mindestens acht Streben aufweist, die sich davon erstrecken,
wobei das sich distal erstreckende Paar von zwei Streben (44) ein erstes sich distal erstreckendes Paar von zwei Streben (44) der mindestens acht Streben ist, und
wobei für jedes Paar von in Umfangsrichtung benachbarten Zwischenknoten (230):
(i) eine dritte sich distal erstreckende Strebe (247a), die sich von dem einen Zwischenknoten (230a) des Paars von Zwischenknoten (230) erstreckt, an der distalen Verbindungsstelle (45) mit (ii) einer vierten sich distal erstreckenden Strebe (247b) konvergiert, die sich von dem anderen Zwischenknoten (230b) des Paars von Zwischenknoten (230) erstreckt,
die dritte sich distal erstreckende Strebe (247a) distal zu der ersten sich distal erstreckenden Strebe (44a) liegt, und
die vierte sich distal erstreckende Strebe (247b) distal zu der zweiten sich distal erstreckenden Strebe (44b) liegt.

13. Herzklappenprothese nach einem der Ansprüche 1-12,
wobei der Klappenprothesenrahmen ein innerer Rahmen (24, 224) ist, der die Mittellängsachse der Herzklappenprothese (22, 222) umgibt, um das Lumen entlang der Achse zu definieren, wenn sich die Herzklappenprothese (22, 222) in dem ausgedehnten Zustand befindet, und
wobei die Herzklappenprothese (22, 222) eine Klappenprothesenrahmenanordnung (21) umfasst, die den inneren Rahmen (24, 224) und einen äußeren Rahmen (80, 280, 380) umfasst, der mit dem inneren Rahmen (24, 224) gekoppelt ist und diesen umgibt.

## Revendications

1. Prothèse valvulaire cardiaque (22, 222) conçue pour une implantation transcathéter, la prothèse valvulaire cardiaque (22, 222) comprenant :
un cadre de prothèse valvulaire qui entoure un axe longitudinal central de la prothèse valvulaire cardiaque (22, 222) de manière à définir une lumière le long de l'axe lorsque la prothèse valvulaire cardiaque (22, 222) se trouve dans un état dilaté ; et
une pluralité de feuillets prothétiques (140), disposés à l'intérieur de la lumière, et agencés de manière à faciliter l'écoulement unidirectionnel de fluide de l'entrée vers la sortie à travers la lumière dans une direction proximale vers distale lorsque la prothèse valvulaire cardiaque (22, 222) se trouve dans l'état dilaté,
ledit cadre de prothèse valvulaire :
comprenant une pluralité d'entretoises, et
étant façonné de façon à définir une pluralité de noeuds intermédiaires (30, 230) qui sont des extrémités proximales et distales intermédiaires (26, 28) du cadre de prothèse valvulaire,
une partie de la pluralité d'entretoises étant des entretoises intermédiaires (40),
chaque noeud intermédiaire (30, 230) comportant au moins quatre entretoises s'étendant à partir de celui-ci, lesdites au moins quatre entretoises comprenant (a) une paire s'étendant de manière distale de deux entretoises (44) parmi lesdites au moins quatre entretoises, et (b) une paire s'étendant de manière proximale de deux entretoises (46) parmi lesdites au moins quatre entretoises,
pour chaque paire de noeuds intermédiaires circonférentiellement adjacents (30, 230) :
(i) une première entretoise s'étendant de manière distale (44a) qui s'étend à partir d'un noeud intermédiaire (30a, 230a) de la paire de noeuds intermédiaires (30, 230) convergeant au niveau d'une jonction distale (45) avec (ii) une deuxième entretoise s'étendant de manière distale (44b) qui s'étend à partir de l'autre noeud intermédiaire (30b, 230b) de la paire de noeuds intermédiaires (30, 230),
(i) une première entretoise s'étendant de manière proximale (46a) qui s'étend à partir du premier noeud intermédiaire (30a, 230a) de la paire de noeuds intermédiaires (30, 230) convergeant au niveau d'une jonction proximale (48a) avec (ii) une deuxième entretoise s'étendant de manière proximale (46b) qui s'étend à partir de l'autre noeud intermédiaire (30b, 230b) de la paire de noeuds intermédiaires (30, 230), et
(i) une première entretoise intermédiaire (40a) s'étendant de manière proximale à partir d'un emplacement intermédiaire (39a) le long de la première entretoise s'étendant de manière distale (44a), et (ii) une seconde entretoise intermédiaire (40b) s'étendant de manière proximale à partir d'un emplacement intermédiaire (39b) le long de la deuxième entretoise s'étendant de manière distale (44b), l'emplacement intermédiaire (39a) le long de la première entretoise s'étendant de manière distale (44a) se trouvant entre le premier noeud intermédiaire (30a, 230a) et la jonction distale (45), et l'emplacement intermédiaire (39b) le long de la deuxième entretoise s'étendant de manière distale (44b) se trouvant entre l'autre noeud intermédiaire (30b, 230b) et la jonction distale (45),
lesdites première et seconde entretoises intermédiaires (40a, 40b) convergeant au niveau d'une jonction intermédiaire (42) qui est orientée dans une direction proximale,
ladite jonction intermédiaire (42) étant intermédiaire entre la jonction distale (45) et la jonction proximale (48a), et
ladite jonction proximale (48a), ladite jonction intermédiaire (42) et ladite jonction distale (45) étant circonférentiellement alignées, **caractérisé en ce que** :
chaque noeud intermédiaire (30, 230) comporte au moins six entretoises s'étendant à partir de celui-ci,
la paire s'étendant de manière proximale des deux entretoises (46) définit une première paire s'étendant de manière proximale de deux entretoises (46) parmi lesdites au moins six entretoises,
lesdites au moins six entretoises comprennent une seconde paire s'étendant de manière proximale de deux entretoises (50) parmi lesdites au moins six entretoises,
la jonction proximale (48a) définit une première jonction proximale (48a), et
pour chaque paire de noeuds intermédiaires circonférentiellement adjacents (30, 230) :
(i) une troisième entretoise s'étendant de manière proximale (50a) qui s'étend à partir dudit premier noeud intermédiaire (30a, 230a) de la paire de noeuds intermédiaires (30, 230) converge vers une seconde jonction proximale (48b) avec (ii) une quatrième entretoise s'étendant de manière proximale (50b) qui s'étend à partir dudit autre noeud intermédiaire (30b, 230b) de la paire de noeuds intermédiaires (30, 230),
la troisième entretoise s'étendant de manière proximale (50a) est proximale par rapport à la première entretoise s'étendant de manière proximale (46a),
la quatrième entretoise s'étendant de manière proximale (50b) est proximale par rapport à la deuxième entretoise s'étendant de manière proximale (46b),
la seconde jonction proximale (48b) est proximale par rapport à la première jonction proximale (48a), et
la seconde jonction proximale (48b) et la première jonction proximale (48a) sont circonférentiellement alignées.

2. Prothèse valvulaire cardiaque selon la revendication 1, lorsque la prothèse valvulaire cardiaque (22, 222) se trouve dans l'état déployé, pour chaque paire de noeuds intermédiaires circonférentiellement adjacents (30, 230) :
un losange plus petit (10) du cadre de prothèse valvulaire étant délimité par (1) des parties distales des première et deuxième entretoises s'étendant de manière distale (44a, 44b), et (2) les première et seconde entretoises intermédiaires (40a, 40b), et
un losange plus grand (12) du cadre de prothèse valvulaire étant délimité par (1) les première et deuxième entretoises s'étendant de manière distale (44a, 44b), et (2) les première et deuxième entretoises s'étendant de manière proximale (46a, 46b),
ledit losange plus petit (10) se trouvant à l'intérieur du losange plus grand (12).

3. Prothèse valvulaire cardiaque selon la revendication 1, lesdites première et seconde entretoises intermédiaires (40a, 40b) comportant des parties longitudinales séquentielles respectives, et lorsque la prothèse valvulaire cardiaque (22, 222) se trouve dans l'état déployé, lesdites parties longitudinales séquentielles respectives :
s'étendant suivant un angle de 75 à 105 degrés à partir des côtés amont respectifs des première et deuxième entretoises s'étendant de manière distale (44a, 44b), respectivement,
se courbant partiellement en amont et partiellement vers les noeuds intermédiaires respectifs les plus proches (30a, 230a, 30b, 230b) le long des premières parties longitudinales incurvées respectives (41) des première et seconde entretoises intermédiaires (40a, 40b), et
se courbant partiellement en amont et partiellement à l'écart des noeuds intermédiaires respectifs les plus proches (30a, 230a, 30b, 230b) le long des secondes parties longitudinales incurvées respectives (43) des première et seconde entretoises intermédiaires (40a, 40b).

4. Prothèse valvulaire cardiaque selon la revendication 3, chacune des premières parties longitudinales incurvées (41) comportant une longueur de 0,2 à 0,75 mm, et chacune des secondes parties longitudinales incurvées (43) comportant une longueur de 1 à 1,75 mm, lesdites longueurs étant mesurées le long des axes longitudinaux centraux incurvés respectifs des parties longitudinales incurvées (41, 43).

5. Prothèse valvulaire cardiaque selon la revendication 3,
chacune des premières parties longitudinales incurvées (41) comportant une première longueur et chacune des secondes parties longitudinales incurvées (43) comportant une seconde longueur, lesdites longueurs étant mesurées le long des axes longitudinaux centraux incurvés respectifs des parties longitudinales incurvées (41, 43), et
un rapport entre la seconde longueur et la première longueur valant de 2 à 8.

6. Prothèse valvulaire cardiaque selon la revendication 3, chacune des premières parties longitudinales incurvées (41) comportant un premier rayon de courbure interne de 0,05 à 0,2 mm, et chacune des secondes parties longitudinales incurvées (43) comportant un second rayon de courbure interne de 0,4 à 0,8 mm.

7. Prothèse valvulaire cardiaque selon la revendication 3,
chacune des premières parties longitudinales incurvées (41) comportant un premier rayon de courbure interne et chacune des secondes parties longitudinales incurvées (43) comportant un second rayon de courbure interne, et
un rapport entre le second rayon de courbure interne et le premier rayon de courbure interne valant de 2 à 10.

8. Prothèse valvulaire cardiaque selon la revendication 1, ledit cadre de prothèse valvulaire comportant une hauteur totale de 20 à 26 mm lorsque la prothèse valvulaire cardiaque (22, 222) se trouve dans l'état déployé.

9. Prothèse valvulaire cardiaque selon l'une quelconque des revendications 1 à 8, lorsque la prothèse valvulaire cardiaque (22, 222) se trouve dans l'état déployé, pour chaque paire de noeuds intermédiaires circonférentiellement adjacents (30, 230) :
un losange plus petit (10) du cadre de prothèse valvulaire étant délimité par (1) les parties distales des première et deuxième entretoises s'étendant de manière distale (44a, 44b), et (2) les première et seconde entretoises intermédiaires (40a, 40b),
un losange plus grand (12) du cadre de prothèse valvulaire étant délimité par (1) les première et deuxième entretoises s'étendant de manière distale (44a, 44b), et (2) les première et deuxième entretoises s'étendant de manière proximale (46a, 46b), et
un plus grand losange (14) du cadre de prothèse valvulaire étant délimité par (1) les première et deuxième entretoises s'étendant de manière distale (44a, 44b), et (2) les troisième et quatrième entretoises s'étendant de manière proximale (50a, 50b),
ledit losange plus petit (10) se trouvant à l'intérieur du losange plus grand (12), et ledit losange plus grand (12) se trouvant à l'intérieur du plus grand losange (14).

10. Prothèse valvulaire cardiaque selon l'une quelconque des revendications 1 à 8,
ladite partie de la pluralité d'entretoises comprenant une première partie de la pluralité d'entretoises,
ledit cadre de prothèse valvulaire comprenant une seconde partie de la pluralité d'entretoises,
ladite seconde partie de la pluralité d'entretoises étant des entretoises proximales (52), et
pour chaque noeud intermédiaire (30, 230) :
(i) une première entretoise proximale (52a) s'étendant de manière proximale à partir d'un emplacement intermédiaire (53a) le long d'une première entretoise (50') de la seconde paire s'étendant de manière proximale des deux entretoises (50) parmi lesdites au moins six entretoises, et (ii) une seconde entretoise proximale (52b) s'étendant de manière proximale à partir d'un emplacement intermédiaire (53b) le long d'une seconde entretoise (50") de la seconde paire s'étendant de manière proximale des deux entretoises (50) parmi lesdites au moins six entretoises, chaque emplacement intermédiaire (53a, 53b) le long des première et seconde entretoises respectives (50', 50") de la seconde paire s'étendant de manière proximale de deux entretoises (50) se trouvant entre le noeud intermédiaire (30, 230) et les extrémités proximales respectives de chacune des première et seconde entretoises (50', 50") de la seconde paire de deux entretoises s'étendant de manière proximale (50),
lesdites première et seconde entretoises proximales (52a, 52b) convergeant au niveau d'une jonction orientée de manière proximale (54) des première et seconde entretoises proximales (52a, 52b),
ladite jonction orientée de manière proximale (54) des première et secondes entretoises proximales (52a, 52b) étant circonférentiellement alignée avec le noeud intermédiaire (30, 230), et ladite jonction orientée de manière proximale (54) étant circonférentiellement décalée par rapport à la seconde jonction proximale (48b).

11. Prothèse valvulaire cardiaque selon la revendication 10, lesdites jonctions orientées de manière proximale (54) étant alignées de manière longitudinale avec les secondes jonctions proximales (48b) au niveau de l'extrémité proximale (26) du cadre de prothèse valvulaire.

12. Prothèse valvulaire cardiaque (222) selon l'une quelconque des revendications 1 à 8,
chaque noeud intermédiaire (230) comportant au moins huit entretoises s'étendant à partir de celui-ci,
ladite paire de deux entretoises s'étendant de manière distale (44) étant une première paire de deux entretoises s'étendant de manière distale (44) parmi lesdites au moins huit entretoises, et
pour chaque paire de noeuds intermédiaires circonférentiellement adjacents (230) :
(i) une troisième entretoise s'étendant de manière distale (247a) qui s'étend à partir du premier noeud intermédiaire (230a) de la paire de noeuds intermédiaires (230) convergeant au niveau de la jonction distale (45) avec (ii) une quatrième entretoise s'étendant de manière distale (247b) qui s'étend à partir de l'autre noeud intermédiaire (230b) de la paire de noeuds intermédiaires (230),
ladite troisième entretoise s'étendant de manière distale (247a) étant distale par rapport à la première entretoise s'étendant de manière distale (44a), et
ladite quatrième entretoise s'étendant de manière distale (247b) étant distale par rapport à la deuxième entretoise s'étendant de manière distale (44b).

13. Prothèse valvulaire cardiaque selon l'une quelconque des revendications 1 à 12,
ledit cadre de prothèse valvulaire étant un cadre interne (24, 224) qui entoure l'axe longitudinal central de la prothèse valvulaire cardiaque (22, 222) de manière à définir la lumière le long de l'axe lorsque la prothèse valvulaire cardiaque (22, 222) se trouve dans l'état déployé, et
ladite prothèse valvulaire cardiaque (22, 222) comprenant un ensemble de cadre de prothèse valvulaire (21) comprenant le cadre interne (24, 224) et un cadre externe (80, 280, 380) qui est couplé au cadre interne (24, 224) et l'entoure.
